(19) Europäisches Patentamt European Patent Office Office européen des brevets

(11) **EP 3 349 775 B1**

(12) **EUROPEAN PATENT SPECIFICATION**

(45) Date of publication and mention of the grant of the patent:
**01.12.2021 Bulletin 2021/48**

(51) Int Cl.:
***C07K 1/04*** *(2006.01)*

(21) Application number: **16847410.4**

(22) Date of filing: **16.09.2016**

(86) International application number:
**PCT/US2016/052179**

(87) International publication number:
**WO 2017/049115 (23.03.2017 Gazette 2017/12)**

(54) **SOLID PHASE PEPTIDE SYNTHESIS METHODS AND ASSOCIATED SYSTEMS**

FESTPHASEN-PEPTIDSYNTHESEVERFAHREN UND ZUGEHÖRIGE SYSTEME

PROCÉDÉS DE SYNTHÈSE DE PEPTIDES EN PHASE SOLIDE ET SYSTÈMES ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2015 US 201562220232 P**

(43) Date of publication of application:
**25.07.2018 Bulletin 2018/30**

(60) Divisional application:
**21203130.6**

(73) Proprietor: **Massachusetts Institute of Technology Cambridge, MA 02139 (US)**

(72) Inventors:
• **THOMAS, Dale Arlington, III**
  **Hampden, ME 04444 (US)**
• **MIJALIS, Alexander James**
  **Shreveport, LA 71106 (US)**
• **PENTELUTE, Bradley L.**
  **Cambridge, MA 02139 (US)**
• **SIMON, Mark David**
  **Gainesville, FL 32607 (US)**
• **ADAMO, Andrea**
  **Cambridge, MA 02139 (US)**
• **HEIDER, Patrick Louis**
  **Midland, MI 48642 (US)**
• **JENSEN, Klavs,F.**
  **Lexington, MA 02421 (US)**

(74) Representative: **Hoffmann Eitle Patent- und Rechtsanwälte PartmbB Arabellastraße 30 81925 München (DE)**

(56) References cited:
| | |
|---|---|
| WO-A1-98/34633 | WO-A2-2014/149387 |
| US-A- 4 816 513 | US-A- 5 807 525 |
| US-A- 6 028 172 | US-A1- 2014 275 481 |

## Description

## TECHNICAL FIELD

Methods and systems for performing solid phase peptide synthesis are generally described. The present invention is as defined in the claims.

## BACKGROUND

Solid phase peptide synthesis is a process used to chemically synthesize peptides on solid supports. In solid phase peptide synthesis, an amino acid or peptide is bound, usually via the C-terminus, to a solid support. New amino acids are added to the bound amino acid or peptide via coupling reactions. Due to the possibility of unintended reactions, protecting groups are typically used. Solid phase peptide synthesis has become standard practice for chemical peptide synthesis. The broad utility of solid phase peptide synthesis has been demonstrated by the commercial success of automated solid phase peptide synthesizers. Though solid phase peptide synthesis has been used for over 30 years, automated solid phase peptide synthesizers that afford a high degree of control over individual coupling reactions and/or minimize side reactions have not yet been developed. Accordingly, improved processes and systems are needed.

WO 2014/149387 A2 relates to a process for adding amino acid residues to peptides, comprising: heating a stream comprising amino acids such that the temperature of the amino acids is increased by at least about 1 °C; and exposing the heated amino acids to a plurality of peptides immobilized on a solid support, wherein the heating step is performed prior to and within about 30 seconds of exposing the heated amino acids to the peptides.

## SUMMARY

Solid phase peptide synthesis methods and associated systems are generally described. Certain embodiments relate to systems and methods for activation of amino acids. In some embodiments, activation reagents can combined in ways that reduce the amount of side reactions and increase yield. The scope of the invention is defined by the appended claims.

In one set of embodiments, methods are provided. In one embodiment of the disclosure, a method of operating a peptide synthesis system comprises flowing a first fluid stream comprising amino acids, flowing a second fluid stream comprising a base, merging the first and second fluid streams at a mixing region to form a mixed fluid stream, and flowing the mixed fluid stream to a reactor. In such embodiments, the molar ratio of the amino acids to the base in the mixed fluid stream at the mixing region is within 10% of a molar ratio of the amino acids to the base at the reactor.

In another embodiment of the disclosure, a method of operating a peptide synthesis system comprises flowing a first fluid stream comprising activating agent, flowing a second fluid stream comprising a base, merging the first and second fluid streams at a mixing region to form a mixed fluid stream, and flowing the mixed fluid stream to a reactor. In such embodiments, the molar ratio of the base to the activating agent in the mixed fluid stream at the mixing region is within 10% of a molar ratio of the base to the activating agent at the reactor.

In one embodiment of the disclosure, a method of operating a peptide synthesis system comprises, flowing a first fluid stream comprising amino acids, flowing a second fluid stream comprising a base, merging the first and second fluid streams at a mixing region to form a mixed fluid stream, and flowing the mixed fluid stream to a reactor. In such embodiments, for a period beginning at a point in time when at least one of an amino acid and a base initially reaches the reactor, a molar ratio of the amino acids to the base in the mixed fluid stream at the mixing region is within 10% of a molar ratio of the amino acids to the base at the reactor.

In another embodiment of the disclosure, a method of operating a peptide synthesis system comprises flowing a first fluid stream comprising activating agent, flowing a second fluid stream comprising a base, merging the first and second fluid streams at a mixing region to form a mixed fluid stream, and flowing the mixed fluid stream to a reactor. In such embodiments, for a period beginning at a point in time when at least one of a base and an activating agent initially reaches the reactor, a molar ratio of the base to the activating agent in the mixed fluid stream at the mixing region is within 10% of a molar ratio of the base to the activating agent at the reactor.

In one embodiment of the disclosure, a method of initiating operation of a peptide synthesis system comprises flowing a first fluid stream comprising amino acids to a mixing region, flowing a second fluid stream comprising a base to the mixing region, merging the first and second fluid streams at a mixing region to form a mixed fluid stream having a leading edge, and flowing the mixed fluid stream to a reactor. In such embodiments, the molar ratio of the amino acids to the base measured at the leading edge as the leading edge enters the reactor is within 10% of a molar ratio of the amino acids to the base in the mixed fluid stream at the mixing region.

In another embodiment of the disclosure, a method of initiating operation of a peptide synthesis system comprises flowing a first fluid stream comprising activating agent to a mixing region, flowing a second fluid stream comprising a base to the mixing region, merging the first and second fluid streams at a mixing region to form a mixed fluid stream having a leading edge, and flowing the mixed fluid stream to a reactor. In such embodiments, the molar ratio of the base to the activating agent measured at the leading edge as the leading edge enters the reactor is within 10% of a molar ratio of the base to the activating agent in the mixed fluid stream at the mixing region.

In an embodiment of the present invention, a method of initiating operation of a peptide synthesis system comprises commencing flow of a first fluid stream comprising amino acids from a first reagent reservoir to a mixing region, commencing flow of a second fluid stream comprising a base from a second reagent reservoir to the mixing region, such that a leading edge of the first fluid stream and a leading edge of the second fluid stream arrive at the mixing region within about 10 ms of each other, merging the first and second fluid streams at a mixing region to form a mixed fluid stream, and flowing the mixed fluid stream to a reactor.

In an embodiment of the present invention, a method of initiating operation of a peptide synthesis system comprises commencing flow of a first fluid stream comprising activating agent from a first reagent reservoir to a mixing region, commencing flow of a second fluid stream comprising a base from a second reagent reservoir to the mixing region, such that a leading edge of the first fluid stream and a leading edge of the second fluid stream arrive at the mixing region within about 10 ms of each other, merging the first and second fluid streams at a mixing region to form a mixed fluid stream, and flowing the mixed fluid stream to a reactor.

In one embodiment of the disclosure, a method of operating a peptide synthesis system, comprising: merging a first fluid stream comprising amino acids and a second fluid stream comprising a base at a junction to form a mixed fluid stream, and flowing the mixed fluid stream from the junction to a reactor and introducing the mixed fluid stream into the reactor, wherein the residence time of the mixed fluid stream from the junction to the reactor is at least about 0.1 seconds and less than about 30 seconds, and wherein the molar ratio of the amino acids to the base in the mixed fluid stream changes by no more than 10% from formation of the mixed fluid stream to introduction of the mixed fluid stream into the reactor.

In another embodiment of the disclosure, a method of initiating operation of a peptide synthesis system comprises flowing a first fluid stream comprising amino acids to a mixing region, flowing a second fluid stream comprising a base to the mixing region, merging the first and second fluid streams at a mixing region to form a mixed fluid stream having a leading edge, and flowing the mixed fluid stream to a reactor, wherein a molar ratio of the amino acids to the base measured at the leading edge as the leading edge enters the reactor is within 10% of a molar ratio of the amino acids to the base in the mixed fluid stream at the entrance to the reactor at a time that is at least about 10 ms after the leading edge enters the reactor.

In one embodiment of the disclosure, a method of initiating operation of a peptide synthesis system comprises flowing a first fluid stream comprising activating agent to a mixing region, flowing a second fluid stream comprising a base to the mixing region, merging the first and second fluid streams at a mixing region to form a mixed fluid stream having a leading edge, and flowing the mixed fluid stream to a reactor, wherein a molar ratio of the activating agent to the base measured at the leading edge as the leading edge enters the reactor is within 10% of a molar ratio of the activating agent to the base in the mixed fluid stream at the entrance to the reactor at a time that is at least about 10 ms after the leading edge enters the reactor.

Other advantages and novel features of the present invention will become apparent from the following detailed description of various non-limiting embodiments of the invention when considered in conjunction with the accompanying figures.

## BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:

FIG. 1 is a schematic of solid phase peptide synthesis, according to one set of embodiments;
FIG. 2A is a schematic illustration of a system for performing peptide synthesis, according to one set of embodiments;
FIG. 2B is a schematic illustration of a system for performing peptide synthesis, according to one set of embodiments;
FIG. 3A is, according to certain embodiments, an exemplary schematic diagram of a peptide synthesis system;
FIG. 3B is, according to certain embodiments, chromatograms for a synthesized peptide; and
FIG. 3C is, according to certain embodiments, concentration profiles for two activation reagents;
FIG. 4A is chromatograms for a synthesized peptide, according to one set of embodiments;
FIGs. 4B is a graph of percentage of identified product versus products for a peptide synthesized at various flow rates, according to one set of embodiments;
FIG. 5A is a photograph of the automated flow solid phase synthesizer, according to one set of embodiments;
FIG. 5B is a cycle diagram of a peptide synthesis, according to certain embodiments;
FIG. 5C is a LC-MS chromatogram for the crude product of acyl carrier protein (65-74) synthesis, according to one set of embodiments;

FIG. 5D is a UV absorbance spectrum for one coupling and deprotection cycle, according to one set of embodiments;

FIG. 6A is a LC-MS chromatograph for Growth Hormone Releasing Hormone (GHRH) synthesized via different methods, according to one set of embodiments;

FIG. 6B is a LC-MS chromatograph for Insulin B-chain synthesized using different methods, according to one set of embodiments;

FIG. 6C is a plot of Fmoc deprotection UV data for each cycle of synthesis for GHRH and Insulin B-chain, according to one set of embodiments;

FIG. 7A is a diagram of the heated portion of the automated flow peptide synthesizer, according to one set of embodiments;

FIG. 7B is a diastereomer analysis of model peptide GCF showing a representative sample from flow synthesis using method B (top) and a 50/50 mixture of the authentic Cys diastereomers (bottom), according to one set of embodiments;

FIG. 7C is a graph of the percentage of Cys diastereomer formation as a function of flow rate (ml/min) using method B, according to one set of embodiments;

FIG. 7D is a diastereomer analysis of model peptide FHL showing a representative sample from flow synthesis using method B (top) and a 50/50 mixture of the authentic Cys diastereomers (bottom), according to one set of embodiments; and

FIG. 7E is a graph of the percentage of histidine diastereomer formation as a function of flow rate (ml/min).

## DETAILED DESCRIPTION

Methods and system for solid phase peptide synthesis are provided. Solid phase peptide synthesis is a known process in which amino acid residues are added to peptides that have been immobilized on a solid support. New amino acid residues are added via a coupling reaction between an activated amino acid and an amino acid residue of the immobilized peptide. Amino acids may be activated using, e.g., a base and an activating agent. Certain inventive concepts, described herein, relate to methods and systems for the activation of amino acids. These systems and methods may allow for fewer side reactions and a higher yield compared to conventional activation techniques as well as the customization of the coupling reaction on a residue-by-residue basis without the need for costly and/or complex processes.

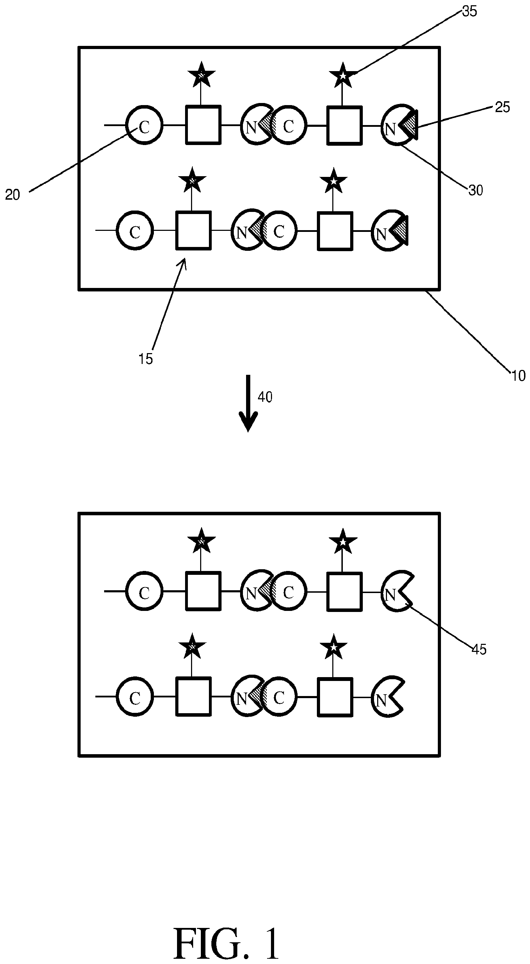

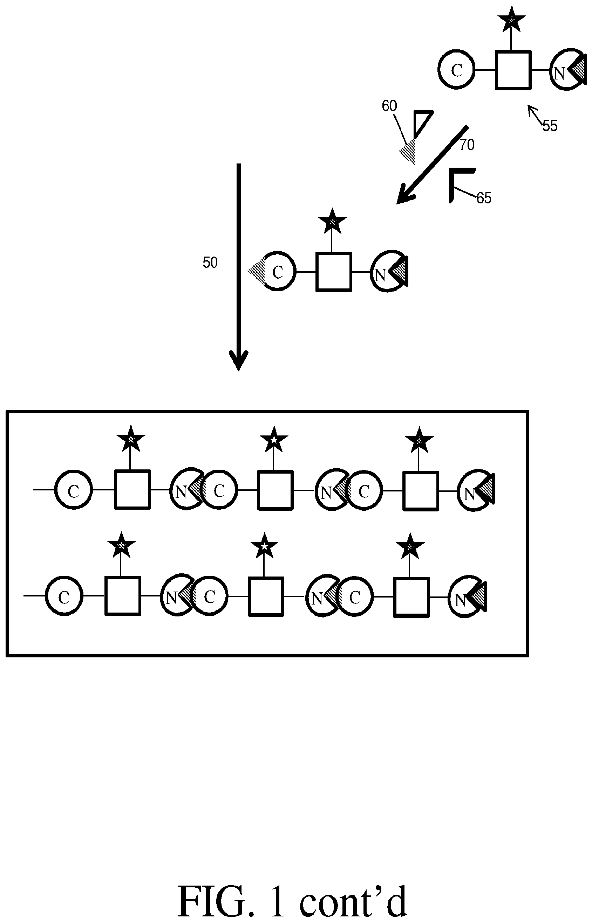

A non-limiting schematic of a solid phase peptide synthesis method is shown in FIG. 1. In some embodiments, a solid phase synthesis method may utilize a solid support 10. Peptides 15 may be bound to the solid support such that each peptide is immobilized on the solid support. For example, the peptides may be bound to the solid support via their C termini 20, thereby immobilizing the peptides. In certain embodiments, peptides 15 may comprise protecting groups 25, for example, on the N-termini 30 of the peptides. In some embodiments, the side chains 35 of the amino acid residues in the peptide may comprise protecting groups as shown in FIG. 1. In some embodiments, the process of adding amino acid residues to immobilized peptides comprises deprotecting at least a portion of the N-termini protecting groups as indicated by arrow 40 to form free N-termini 45 as shown in FIG. 1

In some embodiments, the free N-termini may be exposed to amino acids, such that at least a portion of the free N-termini may undergo a coupling with the C-termini of the amino acids resulting in the formation of an amide bond and the addition of a newly-bonded amino acid residue to the immobilized peptide as indicated by arrow 50. In certain embodiments, the amino acids 55 may be activated prior to exposure to free N-termini 45 as indicated by arrow 70. Activation of the amino acid may facilitate the coupling reaction such that the yield of the coupling reaction is relatively high (e.g., greater than or equal to about 98%).

In general, the activated amino acids may be formed using any suitable reagents. In certain embodiments, activated amino acids may be formed using an activating agent 60 and a base 65 as shown in FIG. 1 and indicated by arrow 70. In some such embodiments, the activated amino acid may not be purified prior to exposure to the immobilized peptides. In such cases, the immobilized peptides may also be exposed to at least a portion of the unreacted activating agent, base, and/or amino acid, some of which may adversely affect peptide synthesis. For example, unreacted uranium or guanidinium activating agent may undergo a coupling reaction with at least a portion of the free N-termini and prevent further addition of amino acid residues. Accordingly, in some embodiments, precise control over the stoichiometric ratio of activation reagents (e.g., activating agent, base, and/or amino acid) is needed to prevent side reaction and thereby increase overall yield.

In some conventional systems to control the stoichiometric ratio, the activation reagents are mixed a long time before exposure to the immobilized peptides and the mixture is stored in the system. In certain embodiments, storage of certain activation reagents together may result in undesirable side reactions prior to exposure to the immobilized peptides. For example, storage of the amino acids with a base may result in degradation, polymerization, protecting group removal, and/or epimerization of the amino acid. In some cases, the yield and kinetics of the coupling reaction are adversely affected. Certain conventional systems have tried to address this problem by mixing activation reagents in the presence of the immobilized peptides. However, this method may result in slower reaction kinetics, truncations of the peptidyl chain, and ultimately lower yields.

Certain inventive methods and systems, described herein, allow for the stoichiometric control of activation reagents with little or no adverse side reactions and/or undesirable impact on yield (e.g., of the coupling reaction, overall). In some embodiments, one or more activation reagents may be stored separately from another activation reagent. For instance, amino acids and/or activating agent may be stored separately (e.g., in reagent reservoirs) from a base. Prior to, but within a short amount of time of, arrival at a reactor, the two or more activation reagents may be mixed, such that the reactor is initially exposed to the two or more activation reagents as a mixture having the desired ratio for activation. In some such embodiments, a first fluid stream comprising a first activation reagent (e.g., amino acids, activating agent) and a second fluid stream comprising a second activation reagent (e.g., base) may be merged at a mixing region (e.g., junction) to form a mixed fluid having a leading edge, which is flowed into the reactor. In some such cases, the molar ratio of the two or more activation reagents (e.g., base and amino acids, base and activating agent) at the mixing region is within 10% (e.g., 5%) of the molar ratio of the two or more activation reagents at the reactor. According to the present invention, the flow of the first and the second fluid streams, at initiation of fluid flow, may be controlled, such that the leading edge of the first fluid stream and the leading edge of the second fluid stream arrive within about 10 ms of each other (e.g., substantially simultaneously). In some embodiments, the first and the second fluid streams may be merged, such that when the leading edge of the mixed fluid enters the reactor, the molar ratio of the two or more activation reagents (e.g., base and amino acids, base and activating agent) at the mixing region is within 10% (e.g., 5%) of the molar ratio of the two or more activation reagents at the reactor. In some embodiments, individual activation reagents may not be introduced to the reactor due to merging at the mixing region.

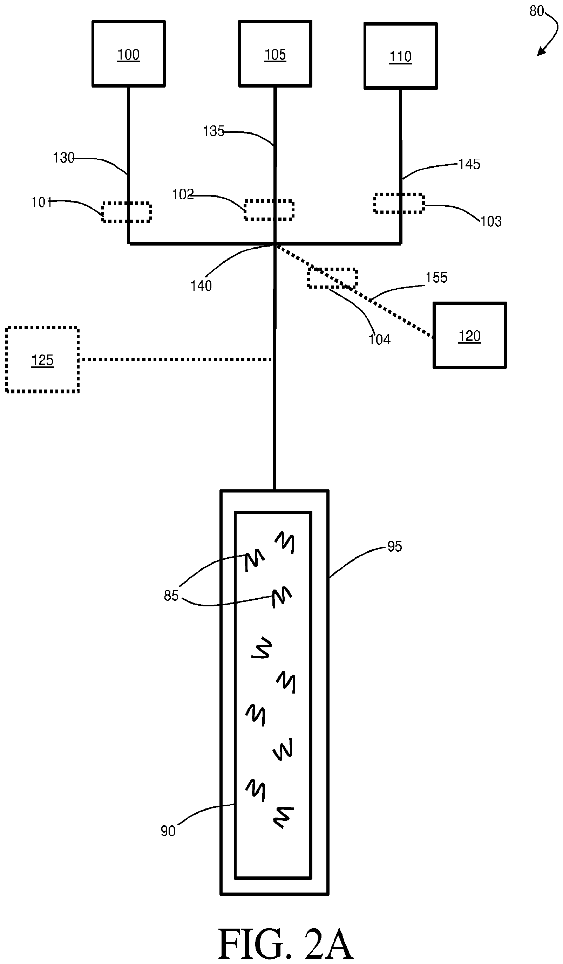

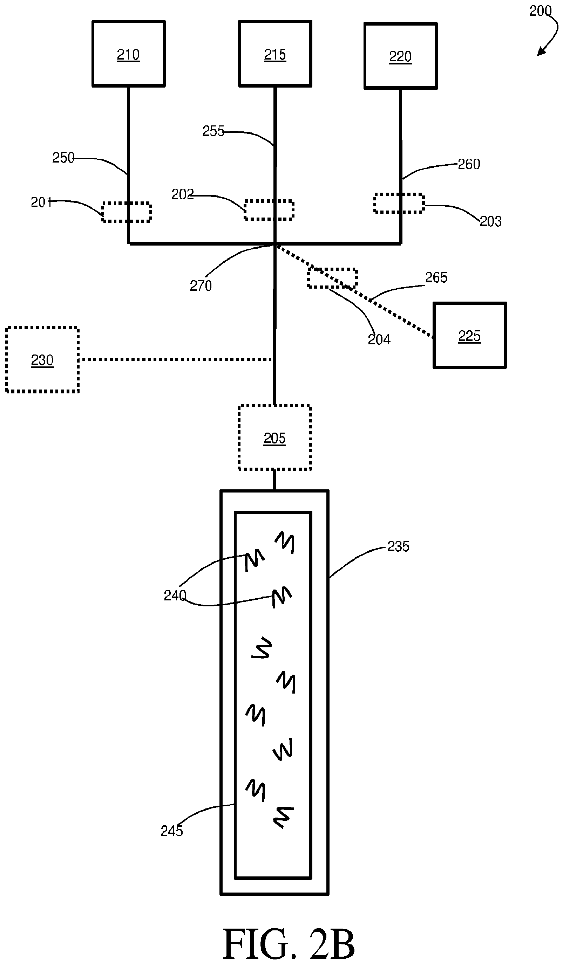

Schematic illustrations of exemplary systems 80 and 200, which can be used to perform certain activation methods described herein are shown in FIGs.2A and 2B. The systems and methods described herein (e.g., system 80 in FIG. 2A, system 200 in FIG. 2B) can involve flow-based synthesis (as opposed to batch-based synthesis, which is employed in many traditional solid phase peptide synthesis systems). In some such embodiments, continuous peptide synthesis can be performed, in which fluid (of one form or another) is substantially continuously transported over the immobilized peptides in a reactor. For example, reagents and rinsing fluids may be alternatively and continuously transported over the immobilized peptides, in certain embodiments.

For instance, in some embodiments, a system may comprise a vessel, such as reactor, that contains peptides and/or amino acids immobilized on a solid support. For example, as shown in FIG. 2A, peptides 85 may be immobilized on a solid support 90. Solid support 90 may be contained within a vessel, such as reactor 95. In some embodiments, and as shown in FIG. 2A, a plurality of reagent reservoirs may be located upstream of and fluidically connected to reactor 95. In some embodiments, a reagent reservoir 100 contains amino acids. In some instances, reagent reservoir 105 contains a base (e.g., diisopropylethylamine). In certain embodiments, reagent reservoir 110 contains an activating agent, such as carbodiimide, guanidinium salt, phosphonium salt, or uronium salt. In some embodiments, system 80 may comprise an optional reagent reservoir 120. In some instances, reagent reservoir 120 may contain one or more additives such as a chaotropic salt, a cosolvent, and/or a surfactant. In certain embodiments, system 80 may contain a second optional reagent reservoir 125. In some instances, reagent reservoir 125 may contain a deprotection reagent, such as piperidine or trifluoroacetic acid, or may contain a solvent, such as dimethylformamide (DMF), that may be used, e.g., in a washing step.

In some embodiments, a system comprises a vessel, such as a reactor, configured to promote and/or facilitate one or more chemical reactions between molecules. For instance, as shown in FIG. 2B, system 200 may comprise reactor 205 configured to promote and/or facilitate one or more chemical reactions between certain reagents and/or reaction products thereof by, e.g., modulating the reaction kinetics and/or reaction time. For example, reactor 205 may be configured to allow the temperature profile of the fluid stream in the reactor to be controlled such that one or more temperature dependent reaction rates can be modulated (e.g., increased, maintained, and/or decreased) to achieve the desired reaction rate(s), reaction product(s), amount of reaction product(s), and/or reaction yield(s). In some embodiments, and as shown in FIG. 2B, a plurality of reagent reservoirs (e.g., 210, 215, 220) may be located upstream of and fluidically connected to reactor 205. For instance, reagent reservoir 210 (e.g., containing amino acids), reagent reservoir 215 (e.g., containing a base), and/or reagent reservoir 220 (e.g., contains an activating agent) may be located upstream of and fluidically connected to reactor 205. In some embodiments, system 200 may comprise one or more optional reagent reservoirs, such reagent reservoir 225 (e.g., containing an additive) and/or reagent reservoir 230 (e.g., containing a deprotection reagent) located upstream of and fluidically connected to reactor 205.

In some embodiments, reactor 205 may configured to promote and/or facilitate a chemical reaction between reagents from one or more reservoirs located upstream of reactor 205, between a reaction product of a reagent and a reagent, and/or between reaction products of reagents. In certain embodiments, reactor 205 may facilitate and/or promote a chemical reaction between two or more reagents from reservoirs located upstream of reactor 205. For instance, reactor 205 may facilitate the deprotonation to of an amino acid (e.g., from reservoir 210) by a base (e.g., from reservoir 215) to produce an amino acid in carboxylate form. In certain embodiments, reactor 205 may promote and/or facilitate a reaction between a reaction product and a reagent from a reservoir located upstream of reactor 205. For instance, reactor 205 may promote the reaction between an amino acid in carboxylate form with an activating agent, e.g., by supplying heat to the reaction. In some such cases, the amino acid in carboxylate form may be formed upstream of reactor 205 (e.g., at or near the mixing region). In other cases, the amino acid in carboxylate form may be formed within reactor 205.

In some embodiments, reactor 205 may be within a heating zone (not shown) or otherwise in communication with a heat source. For example, system 200 may comprise a heating zone (not shown), within which the contents of the fluid stream in reactor 205 may be heated. The heating zone may comprise a heat source, such as a heater. In general, any suitable method of heating may be used to control the temperature of the fluid stream in the reactor. For example, the heating zone may comprise a liquid bath (e.g., a water bath), a resistive heater, a gas convection-based heating element, a microwave heating element, or any other suitable device designed to produce heat upon the application of energy or due to a chemical reaction. In certain embodiments, the mixed fluid stream may not be exposed to a heat source prior to arrival at the reactor. In some embodiments, the mixed fluid stream may not be exposed to heat from a heat source between the mixing region and the entrance to the reactor. In some such cases, the temperature of the mixed fluid stream is within about 10°C (e.g., within about 8°C, within about 5°C, within about 3°C, within about 2°C, within about 1°C) of the temperature of the leading edge at the entrance of the reactor. For example, the temperature of the leading edge may vary by less than or equal to about 10°C (e.g., less than or equal to about 8°C, less than or equal to about 5°C, less than or equal to about 3°C, less than or equal to about 2°C, less than or equal to about 1°C) from the mixing region to the entrance of the reactor.

In some embodiments, system 200 may comprise two or more reactors. For example, as shown in FIG. 2B, system 200 may comprise reactor 205 upstream of reactor 235. In certain embodiments, reactor 205 may not comprise a plurality of amino acids immobilized and/or a plurality of peptides immobilized on a solid support. In some such cases, the formation of one or more amino acid residue may not occur in reactor 205. In some instances, reagents or reaction product thereof may undergo one or more chemical reactions in reactor 205. For example, an amino acid and a base within the mixed fluid stream may react to produce a deprotonated amino acid (e.g., amino acid in carboxylate form) in reactor 205 and/or an amino acid (e.g., amino acid in carboxylate form) and an activation agent within the mixed fluid stream may react to produce an activated amino acid in reactor 205. In some embodiments, the formation of one or more amino acid residue may occur in reactor 235. In some such embodiments, reactor 235 may contain peptides and/or amino acids immobilized on a solid support. For example, as shown in FIG. 2B, peptides 240 may be immobilized on a solid support 245. Solid support 245 may be contained within reactor 235.

In some embodiments, reactor 205 and reactor 235 may be direct fluid communication (e.g., adjacent, directly connected) as shown in FIG. 2B. Direct fluid communication between the reactors may be advantageous. For instance, the direct fluid communication between reactor 205 and reactor 235 may allow the facilitation and/or promotion of a chemical reaction in reactor 205 to occur just prior to the fluid stream being exposed to reactor 235. In embodiments in which facilitation and/or promotion comprising heating the fluid stream, heating the fluid stream in reactor 205 just prior to being exposed to the immobilized peptides (as opposed to heating the stream long before transport of the stream contents to the immobilized peptides) in reactor 235 may minimize the thermal degradation of one or more reagents (such as, for example, the amino acids that are to be added to the peptides and/or the deprotection reagent) in the stream. In some instances, reactor 205 may be within a short distance of the reactor 235, for example, within about 5 meters, within about 1 meter, within about 50 cm, or within about 10 cm.

While single reservoirs have been illustrated in FIGs. 2A and 2B for simplicity, it should be understood that in FIGs. 2A and 2B, where single reservoirs are illustrated, multiple reservoirs (e.g., each containing different types of amino acids, different types of activating agents, different types of bases, different types of additives, etc.) could be used in place of the single reservoir. It should be understood that though the first and the second fluid streams are described as comprising amino acids and a base, respectively, the first and the second fluid streams may comprise any suitable activation reagent. For instance, the first and the second fluid streams, as described herein, may comprise an activating agent and a base, respectively.

According to the present invention, a method for solid phase peptide synthesis comprises commencing flow of a first stream comprising a first activation reagent (e.g. amino acids, activating agent), commencing flow of a second stream comprising a second activation reagent (e.g. base), and merging the first and second fluid streams at a mixing region to form a mixed fluid stream having a leading edge. The mixed fluid stream is flowed to a reactor. In some embodiments, merging may include the meeting and/or combination of the leading edges of two or more fluid streams to form a single mixed fluid stream. For example, referring back to FIGs. 2A and 2B, flow may be commenced from a reagent reservoir (e.g., 100, 210) to form a first fluid stream (e.g., 130, 250) that comprises a first activation reagent (e.g., amino acids, activation agent) and flow may be commenced from another reagent reservoir (e.g., 105, 215) to form a second fluid stream (e.g., 135, 255) that comprises a second activation reagent (e.g., base). The flow of the first and the second streams may be controlled, at initiation of fluid flow from the reservoirs, such that the leading edge of the first fluid stream meets the leading edge of the second fluid stream at a mixing region (e.g., 140, 270). The leading edge of the first fluid stream and the leading edge of the second fluid stream arrive at the mixing region within about 10 ms of one another (e.g., at substantially the same time). In some such cases, the leading edge of the first fluid stream and/or the leading edge of the second fluid stream do not individually flow (i.e. in a non-merged state) downstream of the mixing region (e.g., 140, 270) prior to the merging of streams occurring at the mixing region.

In some embodiments, when the leading edges of the first and the second streams meet or otherwise arrive within about 10 ms of one another at the mixing region (e.g., 140, 270), the molar ratio of two or more activation reagents (e.g., amino acids to base, base to activation agent) may be substantially the same as the desired ratio for amino acid activation and/or the desired ratio to prevent side reactions of the amino acid and/or immobilized peptides in the reactor (e.g., during coupling). In some such cases, the molar ratio of the first activation reagent (e.g., amino acids, activating agent) to the second activating agent (e.g., base) in the mixed fluid stream does not significantly changes (e.g., by no more than 10%, by no more than 5%) from when the mixed fluid stream is formed at the mixing region from the leading edges and/or from slightly offset (e.g., offset by less than about 10 ms) leading edges of two or more activation reagent fluid stream to the introduction of the mixed fluid stream into the reactor. In some such embodiments, when at least one of the first activation reagent and the second activation reagent (e.g., at least one of amino acids and a base, at least one of an activating agent and a base) initially reaches the reactor, the molar ratio of the first activation reagent (e.g., amino acids, activating agent) to the second activation reagent (e.g., base) in the mixed fluid stream at the mixing region is within 10% (e.g., 5%) of a molar ratio of the first activation reagent to the second activation reagent at the reactor. For instance, in some embodiments, when the leading edge of the mixed fluid enters the reactors, the molar ratio of the first activation reagent (e.g., amino acids, activating agent) to the second activation reagent (e.g., base) at the leading edge of the mixed fluid is within 10% (e.g., 5%) of a molar ratio of the first activation reagent to the second activation reagent at the mixed fluid at the mixing region. In some embodiments, the molar ratio at the entrance to the reactor may vary by less than about 10% (e.g., 5%) from the time the leading edge enters the reactor to a point in time at least about 10 ms, at least about 50 ms, at least about 100 ms, or at least about 1 second later.

In some embodiments, the molar ratio of the first activation reagent (e.g., amino acids, activating agent) to the second activating agent (e.g., base) in the mixed fluid stream at the mixing region does not significantly changes (e.g., by no more than 10%, by no more than 5%) from when the mixed fluid stream is formed at the mixing region from the leading edges and/or from slightly offset (e.g., offset by less than about 10 ms) leading edges of two or more activation reagent fluid stream to a point later in time (e.g., at least about 10 ms, at least about 50 ms, at least about 100 ms, or at least about 1 second later). For instance, the molar ratio of the first activation reagent to the second activating agent at the mixing region when the mixed fluid stream is formed at the mixing region from the leading edges may be within 10% (e.g., 5%, 2%, 1%, 0.5%) of the molar ratio at the mixing region after at least about 10 ms (e.g., at least about 50 ms, at least about 100 ms, or at least about 1 second) after formation of the mixed fluid stream.

In certain embodiments, the first or second fluid stream may also comprise another activation reagent. For instance, the second fluid stream may comprise a base and an activating agent. In some such embodiments, the mixed fluid stream may comprise activated amino acids due to the activation of the amino acids by the base and the activating agent. In some such cases, the mixed fluid stream may not comprise excess base and/or activating agent.

In some embodiments, the first or second fluid stream may not comprise another activation reagent. In some such embodiments, a method for solid phase peptide synthesis may comprise flowing a first stream comprising amino acids, flowing a second stream comprising a base, flowing a third stream comprising an activating agent, and merging the fluid streams at a mixing region to form a mixed fluid stream. The mixed fluid stream may be flowed to a reactor. In some such embodiments, merging may include the meeting and/or combination of the leading edges of three or more fluid streams (e.g., leading edges of the first, second, and third fluid streams) to form a single mixed fluid stream having a leading edge. For example, referring to FIGs. 2A and 2B, flow may be commenced from a reservoir (e.g., 100, 210) that comprises amino acids to form a first stream (e.g., 130, 250), flow may be commenced from another reservoir (e.g., 105, 215) that comprises a base to form a second stream (e.g., 135, 255), and flow may be commenced from a different reservoir (e.g., 110, 220) to form a third stream (e.g., 145, 260). In some embodiments, when the leading edges of the three fluid streams meet or otherwise arrive within about 10 ms of one another at a mixing region (e.g., 140, 270), two or more molar ratios of activation reagents (e.g., amino acids to base and/or base to activation agent) may be substantially the same as the desired ratio for amino acid activation and/or the desired ratio to prevent side reactions of the amino acid and/or immobilized peptides in the reactor (e.g., during coupling). In some such cases, the two or more molar ratios (e.g., first activation reagent to the second activating agent, first activation reagent to the third activating agent, and/or second activation reagent to the third activating agent) in the mixed fluid stream do not significantly changes (e.g., by no more than 10%, by no more than 5%) from when the mixed fluid stream is formed at the mixing region from the leading edges and/or from slightly offset (e.g., offset by less than about 10 ms) leading edges of three or more activation reagent fluid stream to the introduction of the mixed fluid stream into the reactor. In some such embodiments, when at least one of (e.g., at least two of) the first activation reagent, second activation reagent, and the third activation reagent initially reaches the reactor, the molar ratios of the first activation reagent (e.g., amino acids) to the second activation reagent (e.g., base), the first activation reagent (e.g., amino acids) to the third activation reagent (e.g., activating agent), and/or the second activation reagent (e.g., base) to the third activation reagent (e.g., activating agent), in the mixed fluid stream at the mixing region is within 10% (e.g., 5%) of the molar ratio(s) at the reactor. For instance, in some embodiments, when the leading edge of the mixed fluid enters the reactors, the molar ratio(s) at the leading edge of the mixed fluid is within 10% (e.g., 5%) of the molar ratio(s) at the mixing region. In some embodiments, the molar ratio(s) at the entrance to the reactor may vary by less than about 10% (e.g., 5%) from the time the leading edge enters the reactor to a point in time at least about 10 ms, at least about 50 ms, at least about 100 ms, or at least about 1 second later.

In some embodiments, the two or more molar ratios (e.g., first activation reagent to the second activating agent, first activation reagent to the third activating agent, and/or second activation reagent to the third activating agent) in the mixed fluid stream at the mixing region do not significantly changes (e.g., by no more than 10%, by no more than 5%) from when the mixed fluid stream is formed at the mixing region from the leading edges and/or from slightly offset (e.g., offset by less than about 10 ms) leading edges of three or more activation reagent fluid stream to a point later in time (e.g., at least about 10 ms, at least about 50 ms, at least about 100 ms, or at least about 1 second later). For instance, the molar ratios at the mixing region when the mixed fluid stream is formed at the mixing region from the leading edges may be within 10% (e.g., 5%, 2%, 1%, 0.5%) of the molar ratio at the mixing region after at least about 10 ms (e.g., at least about 50 ms, at least about 100 ms, or at least about 1 second) after formation of the mixed fluid stream.

In certain embodiments, a method for solid phase peptide synthesis may comprises merging a fourth fluid stream comprising one or more additives with one or more of the first, second, and third fluid streams at a mixing region (e.g., junction) to form a mixed fluid stream as described above. For example, referring to FIGs. 2A and 2B, flow may be commenced from a optional reservoir (e.g., 120, 225) that comprises one or more additives to form the fourth fluid stream (e.g., 155, 265) to the mixing region (e.g., 140, 270). In some instances, the leading edge of the fourth fluid stream may be merged with leading edges of the first, second, and third fluid streams at the mixing region (e.g., junction). In such cases, the molar ratios of the activation reagents (e.g., amino acid, activating agent, base) may not be substantially changed (by no more than 5%) by the addition of the fourth fluid stream, as described above.

During the merging process, the leading edges of two or more fluid streams (e.g., first and second fluid streams; first, second, and third fluid streams; first, second, third, and fourth fluid streams, all) arrive at the mixing region within about 10 ms of each other. In some embodiments, the time within which the fluid streams arrive at the mixing region may be determined using a UV-vis detector positioned on each activation reagent fluid stream adjacent to the mixing region and a UV-vis detector positioned downstream of and adjacent to the mixing region. Upon commencement of fluid flow, the detectors take continual measurements over time until a signal indicative of the mixed fluid is measured at the UV-vis detector positioned downstream of the mixing region. The curves of absorbance versus time generated from the UV-vis measurements are overlaid with one another. The difference in time between detection of the fluid is used to determine the time within which two or more fluids arrive at the mixing region. It should be noted that each UV-vis detector is set to the appropriate wavelength to measure the relevant fluid stream.

In certain embodiments, after the fluid streams have been merged, the reactor and/or the immobilized peptides may be exposed to the mixed fluid stream within a relatively short period of time. For example, in certain embodiments, the reactor and/or the peptides immobilized on the solid support may be exposed to the mixed fluid within about 30 seconds (or within about 15 seconds, within about 10 seconds, within about 5 seconds, within about 3 seconds, within about 2 seconds, within about 1 second, within about 0.1 seconds, or within about 0.01 seconds) after merging the fluid streams (e.g., first and second fluid streams; first, second, and third fluid streams; first, second, third, and fourth fluid streams) to form the mixed fluid stream. In some embodiments, the residence time of the mixed fluid stream from the mixing region (e.g., junction) to the reactor is at least about 0.1 seconds and less than about 30 seconds, least about 0.1 seconds and less than about 25 seconds, least about 0.1 seconds and less than about 20 seconds, least about 0.1 seconds and less than about 15 seconds, least about 0.1 seconds and less than about 10 seconds, least about 1 second and less than about 30 seconds, least about 1 second and less than about 25 seconds, or least about 1 second and less than about 15 seconds. A used herein, the residence time refers to the total time required for a fluid stream to travel from the mixing region to the entrance of the reactor.

In certain embodiments, after the fluid streams have been merged, but prior to introduction into a reactor, at least a portion of the mixed fluid stream may be flowed into a mixer positioned between the mixing region (e.g., junction) and the reactor. The mixer may facilitate the formation of a homogeneous fluid stream by promoting active and/or passive mixing. In general, any suitable mixer may be used and those of ordinary skill in the art would be knowledgeable of active mixers and passive mixers.

In some embodiments, the difference in the molar ratio between two activation reagents (e.g., amino acids to base, base to activating agent, amino acids to activating agent) at the mixing region and the same molar ratio at the reactor and/or the difference in the molar ratio between two activation reagents at the mixing region at a first time and the same molar ratio at the mixing region at a later point in time (e.g., second time) is less than or equal to about 10%, less than or equal to about 8%, less than or equal to about 6%, less than or equal to about 5%, less than or equal to about 4%, less than or equal to about 2%, less than or equal to about 1%, less than or equal to about 10%, less than or equal to about 10%, or less than or equal to about 0.5%. For instance, in some embodiments, the molar ratio between two activation reagents (e.g., amino acids to base, base to activating agent, amino acids to activating agent) changes by no more than 10% (e.g., no more than 8%, no more than 6%, no more than 5%, no more than 4% , no more than 2%, no more than 1%, no more than 0.5%) from when the mixed fluid stream is formed at the mixing region from the leading edges and/or from slightly offset (e.g., offset by less than about 10 ms) leading edges to introduction of the mixed fluid stream into the reactor and/or from a first point in time at the mixing region to a later point in time at the mixing region.

In some embodiments, the molar ratio at the entrance to the reactor may vary by less than about less than or equal to about 10%, less than or equal to about 8%, less than or equal to about 6%, less than or equal to about 5%, less than or equal to about 4%, less than or equal to about 2%, less than or equal to about 1%, less than or equal to about 10%, less than or equal to about 10%, or less than or equal to about 0.5% from the time the leading edge enters the reactor to a point in time at least about 10 ms, at least about 15 ms, at least about 25 ms, at least about 50 ms, at least about 75 ms, at least about 100 ms, or at least about 1 second later.

When calculating the percentage difference between two values (unless specified otherwise herein), the percentage calculation is made using the value that is larger in magnitude as the basis. To illustrate, if a first value is $V_1$, and a second value is $V_2$ (which is larger than $V_1$), the percentage difference ($V\%_{Diff}$) between $V_1$ and $V_2$ would be calculated as:

$$V_{\%Diff} = \frac{V_2 - V_1}{V_2} \times 100\%$$

The first and second values would be said to be within X% of each other if $V_{\%Diff}$ is less than X%. The first and second values would be said to be at least X% different if $V_{\%Diff}$ is X% or more.

In some embodiments, the molar ratio of one to another activation reagent (e.g., base to activating agent, amino acids to base, amino acids to activating agent) may be at least about 1:0.7 and less than about 2:1. For instance, the molar ratio of a first activation reagent to a second activation reagent (e.g., amino acids to base, base to activating agent, activating agent to base) may be at least about 1:0.7 and less than about 2:1 (e.g., at least about 1:0.8 and less than about 2:1, at least about 1:0.9 and less than about 2:1, at least about 1:1 and less than about 2:1, at least about 1:0.7 and less than about 1.9:1, at least about 1:0.7 and less than about 1.8:1, at least about 1:0.7 and less than about 1.6:1, at least about 1:0.7 and less than about 1.5:1, at least about 1:0.7 and less than about 1.4:1, between about 1:0.7 and about 1.2:1, between about 1:0.7 and about 1:1). In some embodiments, the molar ratio of a first activation reagent (e.g., activating agent) to a second activation reagent (e.g., base) may be at least 1:1. In some embodiments, molar ratios may be determined using a UV-vis detector positioned on each activation reagent fluid stream adjacent to the mixing reagent, a UV-vis detector positioned downstream of the mixing region, and a UV-vis detector positioned at the entrance to the reactor. Upon commencement of fluid flow, the detector take continual measurements until a signal indicative of the mixed fluid is measured at the UV-vis detector positioned downstream of and adjacent to the mixing region. When a signal indicative of the mixed fluid is measured at the UV-vis detector positioned downstream of the mixing region, UV-vis measurements are taken every 25 ms at the wavelengths needed to determine the concentration of each fluid in the mixed fluid stream. The curves of absorbance versus time generated from the UV-vis measurements are overlaid with one another. The concentration of each activation reagent at each relevant location is determined from the curves.

In general, streams may be merged using any suitable technique known to those of skill in the art. In some embodiments, the streams may be merged by flowing the fluid streams (e.g., first, second, third, and/or fourth) substantially simultaneously into a single stream (e.g., by merging channels through which the streams flow). For example, referring to FIGs. 2A and 2B, optional flow rate controllers (e.g., pump) 101, 102, 103, 104, 201, 202, 203, and 204 may be used to control the flow rate and time of arrival of fluid streams 130, 135, 145, 155, 250, 255, 260, and 265, respectively. At least a portion of the flow controllers may be configured to allow for merging, as described herein. In such cases, pumps may be configured such that dead volumes of the pump heads and upstream fluidics are substantially the same (e.g., identical). In some instances, lengths of 130, 135, 145, 250, 255, or 260 in FIGs. 2A and 2B may be adjusted to correct for differences in internal volume. The pumps are then started within a certain time limit of one another (e.g., within about 100 ms, within about 80 ms, within about 60 ms, within about 50 ms, within about 40 ms, within about 25 ms, within about 10 ms, within about 5 ms). Two or more pumps may be linked together in such a manner.

As described herein, the methods and systems for amino acid activation may be used in solid phase peptide synthesis, which is described in more detail below. In general, solid phase peptide synthesis comprise repeating amino acid addition cycles including a deprotection reaction, a coupling reaction, optional reagent removal (e.g., wash) steps. In some embodiments, merging activation reagent streams may be used in one or more amino acid addition cycle, as described in more detail below, of solid phase peptide synthesis. For example, a first fluid stream comprising amino acids and a second stream comprising a base may be merged to form a mixed fluid stream within about 30 seconds prior to exposing the activated amino acids to peptides immobilized on a solid support. In some embodiments, in which more than one amino acid addition cycle is performed during solid phase peptide synthesis, one or more amino acid addition cycles (e.g., a first and a second amino acid addition cycle) may comprise merging a first fluid stream comprising amino acids and a second stream comprising a base to form a mixed fluid stream within about 30 seconds prior to exposing the amino acids to the solid support.

Exemplary amino acid addition cycles and peptide synthesis are now described in more detail. In some embodiments, the process of adding amino acid residues to immobilized peptides comprises exposing a deprotection reagent to the immobilized peptides to remove at least a portion of the protecting groups from at least a portion of the immobilized peptides. The deprotection reagent exposure step can be configured, in certain embodiments, such that side-chain protecting groups are preserved, while N-terminal protecting groups are removed. For instance, in certain embodiments, the protecting group used to protect the peptides comprises fluorenylmethyloxycarbonyl (Fmoc). In some such embodiments, a deprotection reagent comprising piperidine (e.g., a piperidine solution) may be exposed to the immobilized peptides such that the Fmoc protecting groups are removed from at least a portion of the immobilized peptides. In some embodiments, the protecting group used to protect the peptides comprises tert butyloxycarbonyl (Boc). In some such embodiments, a deprotection reagent comprising trifluoroacetic acid may be exposed to the immobilized peptides such that the Boc protecting groups are removed from at least a portion of the immobilized peptides. In some instances, the protecting groups (e.g., tert-butoxycarbonyl, i.e., Boc) may be bound to the N-termini of the peptides.

In some embodiments, the process of adding amino acid residues to immobilized peptides comprises removing at least a portion of the deprotection reagent. In some embodiments, at least a portion of any reaction byproducts (e.g., removed protecting groups) that may have formed during the deprotection step can be removed. In some instances, the deprotection reagent (and, in certain embodiments byproducts) may be removed by washing the peptides, solid support, and/or surrounding areas with a fluid (e.g., a liquid such as an aqueous or non-aqueous solvent, and a supercritical fluid), for example stored in optional reservoir 125. In some instances, removing the deprotection reagent and reaction byproducts may improve the performance of subsequent steps (e.g., by preventing side reactions). In certain embodiments, the performance of subsequent steps is not dependent on the removal of at least a portion (e.g., substantially all) of the deprotection reagent and/or reaction byproducts. In some such cases, the removal step is optional. In embodiments in which the removal step is optional, the removal step may be reduced (e.g., reduction in time of the removal step, reduction in the amount of solvent used in the removal step) and/or eliminated. The reduction or elimination of one or more removal steps may reduce the overall cycle time. It should be understood that if an optional removal step is reduced or eliminated the subsequent step in the addition cycle may serve to remove at least a portion of the deprotection reagent and/or reaction byproducts, e.g., due to fluid flow in the system.

The process of adding amino acid residues to immobilized peptides comprises, in certain embodiments, exposing activated amino acids to the immobilized peptides such that at least a portion of the activated amino acids are bonded to the immobilized peptides to form newly-bonded amino acid residues. For example, the peptides may be exposed to activated amino acids that react with the deprotected N-termini of the peptides. In certain embodiments, amino acids can be activated for reaction with the deprotected peptides by mixing an amino acid-containing stream with an activation agent stream, as discussed in more detail below. In some instances, the amine group of the activated amino acid may be protected, such that addition of the amino acid results in an immobilized peptide with a protected N-terminus. In some embodiments, the peptides may be exposed to activated amino acids that react with deprotected side chains of the immobilized peptides.

In some embodiments, the process of adding amino acid residues to immobilized peptides comprises removing at least a portion of the activated amino acids that do not bond to the immobilized peptides. In some embodiments, at least a portion of the reaction byproducts that may form during the activated amino acid exposure step may be removed. In some instances, the activated amino acids and byproducts may be removed by washing the peptides, solid support, and/or surrounding areas with a fluid (e.g., a liquid such as an aqueous or non-aqueous solvent, and a supercritical fluid), for example stored in optional reservoir 125. In some instances, removing at least a portion of the activated amino acids and reaction byproducts may improve the performance of subsequent steps (e.g., by preventing side reactions). In certain embodiments, the performance of subsequent steps is not dependent on the removal of at least a portion (e.g., substantially all) of the activated amino acids and/or reaction byproducts. In some such cases, the removal step is optional. In embodiments in which the removal step is optional, the removal step may be reduced (e.g., reduction in time of the removal step, reduction in the amount of solvent used in the removal step) and/or eliminated. The reduction or elimination of one or more removal step may reduce the overall cycle time. It should be understood that if an optional removal step is reduced or eliminated the subsequent step in the addition cycle may serve to remove at least a portion of the activated amino acids and/or reaction byproducts, e.g., due to fluid flow in the system.

It should be understood that the above-referenced steps are exemplary and an amino acid addition cycle need not necessarily comprise all of the above-referenced steps. For example, an amino acid addition cycle may not include the deprotection reagent removal step and/or the activated amino acid removal step. Generally, an amino acid addition cycle includes any series of steps that results in the addition of an amino acid residue to a peptide.

In certain embodiments, during the amino acid addition steps, adding the amino acid can result in the peptide incorporating a single additional amino acid residue (i.e., a single amino acid residue can be added to the immobilized peptides such that a given peptide includes a single additional amino acid residue after the addition step). In some such embodiments, subsequent amino acid addition steps can be used to build peptides by adding amino acid residues individually until the desired peptide has been synthesized. In some embodiments, more than one amino acid residue (e.g., in the form of a peptide) may be added to a peptide immobilized on a solid support (i.e., a peptide comprising multiple amino acid residues can be added to a given immobilized peptide). Addition of peptides to immobilized peptides can be achieved through processes know to those of ordinary skill in the art (e.g., fragment condensation, chemical ligation). That is to say, during the amino acid addition step, adding an amino acid to an immobilized peptide can comprise adding a single amino acid residue to an immobilized peptide or adding a plurality of amino acid residues (e.g., as a peptide) to an immobilized peptide.

In certain embodiments, the first amino acid addition step (and/or subsequent amino acid addition steps) may add amino acids at a relatively high yield. For example, certain embodiments include exposing amino acids to the immobilized peptides such that an amino acid residue is added to at least about 99%, at least about 99.9%, at least about 99.99%, or substantially 100% of the immobilized peptides. In certain embodiments, a second (and, in some embodiments, a third, a fourth, a fifth, and/or a subsequent) amino acid addition cycle can be performed which may include exposing amino acids to the immobilized peptides such that an amino acid residue is added to at least about 99%, at least about 99.9%, at least about 99.99%, or substantially 100% of the immobilized peptides. In certain embodiments, the use of processes and systems of the present invention may allow the addition of more than one amino acid to the immobilized peptides to occur relatively quickly (including within any of the time ranges disclosed above or elsewhere herein), while maintaining a high reaction yield.

In certain embodiments, one or more amino acid addition steps can be performed while little or no double incorporation (i.e., adding multiple copies of a desired amino acid (e.g., single amino acid residues or peptides) during a single addition step). For example, in certain embodiments, multiple copies of the desired amino acid are bonded to fewer than about 1% (or fewer than about 0.1%, fewer than about 0.01%, fewer than about 0.001%, fewer than about 0.0001%, fewer than about 0.00001%, or substantially none) of the immobilized peptides during a first (and/or second, third, fourth, fifth, and/or subsequent) amino acid addition step.

In some embodiments, multiple amino acid addition cycles can be performed. Performing multiple amino acid addition cycles can result in more than one single-amino-acid residue (or more than one peptide, and/or at least one single-amino-acid residue and at least one peptide) being added to a peptide. In certain embodiments a process for adding more than one amino acid to immobilized peptides may comprise performing a first amino acid addition cycle to add a first amino acid and performing a second amino acid addition cycle to add a second amino acid. In certain embodiments, third, fourth, fifth, and subsequent amino acid addition cycles may be performed to produce an immobilized peptide of any desired length. In some embodiments, at least about 10 amino acid addition cycles, at least about 50 amino acid addition cycles, or at least about 100 amino acid addition cycles are performed, resulting in the addition of at least about 10 amino acid residues, at least about 50 amino acid residues, or at least about 100 amino acid residues to the immobilized peptides. In certain such embodiments, a relatively high percentage of the amino acid addition cycles (e.g., at least about 50%, at least about 75%, at least about 90%, at least about 95%, or at least about 99% of such amino acid addition cycles) can be performed at high yield (e.g., at least about 99%, at least about 99.9%, at least about 99.99%, or substantially 100%). In some such embodiments, a relatively high percentage of the amino acid addition cycles (e.g., at least about 50%, at least about 75%, at least about 90%, at least about 95%, or at least about 99% of such amino acid addition cycles) can be performed quickly, for example, within any of the time ranges specified above or elsewhere herein. In some such embodiments, a relatively high percentage of the amino acid addition cycles (e.g., at least about 50%, at least about 75%, at least about 90%, at least about 95%, or at least about 99% of such amino acid addition cycles) can be performed with limited or no double incorporation, for example, within any of the double incorporation ranges specified above or elsewhere herein.

In some embodiments, solid phase peptide synthesis may involve heating a stream prior to, but within a short period of time of, arrival at the reactor. Supplying the reactor with a heated stream may alter the kinetics of a process occurring in the reactor. For example, exposing immobilized peptides, solid supports, or other synthesis components to a heated stream may alter the reaction kinetics and/or diffusion kinetics of the amino acid addition process. For example, exposing the peptides to a heated stream comprising activated amino acids may increase the rate at which amino acids are added to the peptides. In some embodiments, heating the stream prior to, but within a short period of time of arrival at the reactor may substantially reduce or eliminate the need to supply auxiliary heat (i.e., heat that is not from one or more pre-heated streams) to the reactor. In some instances, most or substantially all of the heat supplied to the reactor originates from the pre-heated stream. For example, in some embodiments, the percentage of thermal energy that is used to heat the reactor that originates from the pre-heated stream(s) may be greater than or equal to about 50%, greater than or equal to about 60%, greater than or equal to about 70%, greater than or equal to about 80%, greater than or equal to about 90%, greater than or equal to about 95%, or greater than or equal to about 99%. In some such embodiments, heating the system in this way can reduce the time required to heat the reactor, immobilized peptides, solid support, activated amino acids, deprotection reagents, wash fluids, and/or other synthesis components to a desirable reaction temperature.

In some embodiments, a process for adding amino acid residues to peptides may comprise heating a stream comprising activated amino acids such that the temperature of the activated amino acids is increased by at least about 1 °C (or at least about 2°C, at least about 5°C, at least about 10°C, at least about 25°C, at least about 50°C, and/or less than or equal to about 100°C, and/or less than or equal to about 75°C) prior to the heated amino acids being exposed to the immobilized peptides. In certain embodiments, a stream comprising any other component (e.g., a washing agent, a deprotection agent, or any other components) may be heated such that the temperature of the stream contents is increased by at least about 1 °C (or at least about 2°C, at least about 5°C, at least about 10°C, at least about 25°C, at least about 50°C, and/or less than or equal to about 100°C, and/or less than or equal to about 75°C) prior to the stream contents being exposed to the immobilized peptides. In some instances, the heating step (e.g., the heating of the activated amino acids and/or the heating of any other component within a stream transported to the immobilized peptides) may be performed within about 30 seconds (or within about 15 seconds, within about 10 seconds, within about 5 seconds, within about 3 seconds, within about 2 seconds, within about 1 second, within about 0.1 seconds, or within about 0.01 seconds) of exposing the stream contents (e.g., the heated activated amino acids) to the immobilized peptides. In some such embodiments, such heating may be achieved by heating a location upstream of the immobilized peptides. In some such embodiments, the heating of the amino acids begins at least about 0.1 seconds, at least about 1 second, at least about 5 seconds, or at least about 10 seconds prior to exposure of the amino acids to the immobilized peptides. In certain embodiments, the amino acids are heated by at least about 1 °C (or at least about 2°C, at least about 5°C, at least about 10°C, at least about 25°C, at least about 50°C, and/or less than or equal to about 100°C, and/or less than or equal to about 75°C) at least about 0.1 seconds, at least about 1 second, at least about 5 seconds, or at least about 10 seconds prior to the amino acids being exposed to the immobilized peptides.

In some embodiments, both the heating of the amino acids and the merging of the amino acids with the base and/or activating agent can be performed before and within a relatively short time of the amino acids contacting the immobilized peptides. Heating the amino acids may be performed before, during, and/or after merging the streams.

In general, any protecting group known to those of ordinary skill in the art can be used. Non-limiting examples of protecting groups (e.g., n-terminal protecting groups) include fluorenylmethyloxycarbonyl, tert-butyloxycarbonyl, allyloxycarbonyl (alloc), carboxybenzyl, and photolabile protecting groups. In certain embodiments, immobilized peptides comprise fluorenylmethyloxycarbonyl protecting groups. In some embodiments, immobilized peptides comprise tert-butyloxycarbonyl protecting groups.

As described herein, a base may be used to activate or complete the activation of amino acids prior to exposing the amino acids to immobilized peptides. Any suitable base may be used. In certain embodiments, the base is a Lewis base. In some embodiments, the base is a non-nucleophilic bases, such as triisopropylethylamine, N,N-diisopropylethylamine, certain tertiary amines, or collidine, that are non-reactive to or react slowly with protected peptides to remove protecting groups. In general, the base may have a sufficient pKa to allow for deprotonation of the amino acid carboxylic acid.

As described elsewhere, an activating agent may be used to form a bond with the C-terminus of an amino acid to facilitate the coupling reaction and the formation of an amide bond. The activating agent may be used to form activated amino acids prior to exposing the amino acids to immobilized peptides. Any suitable activating agent may be used. In some embodiments, the activating agent is selected from the group consisting of a carbodiimide, guanidinium salt, phosphonium salt, and uronium salt. The activating agent comprises, in some embodiments, a carbodiimide, such as N,N'-dicyclohexylcarbodiimide (DCC), and 1-ethyl-3-(3-dimethylaminopropyl)carbodiimide (EDC). In certain embodiments, the activating agent comprises a uronium activating agent, such as O-(Benzotriazol-1-yl)-N,N,N',N'-tetramethyluronium hexafluorophosphate (HBTU); 2-(7-Aza-1H-benzotriazole-1-yl)-1,1,3,3-tetramethyluronium hexafluorophosphate (HATU); and 1-[(1-(Cyano-2-ethoxy-2-ox-oethylideneaminooxy) dimethylaminomorpholino)] uro-nium hexafluorophosphate (COMU). In certain embodiments, the activating agent comprises a phosphonium activating agent, such as (Benzotriazol-1-yloxy)tripyrrolidinophosphonium hexafluorophosphate (PyBOP ).

As described elsewhere, peptides may be immobilized on a solid support. In general, any solid support may be used with any of the addition cycles described herein. Non-limiting examples of solid support materials include polystyrene (e.g., in resin form such as microporous polystyrene resin, mesoporous polystyrene resin, macroporous polystyrene resin), glass, polysaccharides (e.g., cellulose, agarose), polyacrylamide resins, polyethylene glycol, or copolymer resins (e.g., comprising polyethylene glycol, polystyrene, etc.).

The solid support may have any suitable form factor. For example, the solid support can be in the form of beads, particles, fibers, or in any other suitable form factor.

In some embodiments, the solid support may be porous. For example, in some embodiments macroporous materials (e.g., macroporous polystyrene resins), mesoporous materials, and/or microporous materials (e.g., microporous polystyrene resin) may be employed as a solid support. The terms "macroporous," "mesoporous," and "microporous," when used in relation to solid supports for peptide synthesis, are known to those of ordinary skill in the art and are used herein in consistent fashion with their description in the International Union of Pure and Applied Chemistry (IUPAC) Compendium of Chemical Terminology, Version 2.3.2, August 19, 2012 (informally known as the "Gold Book"). Generally, microporous materials include those having pores with cross-sectional diameters of less than about 2 nanometers. Mesoporous materials include those having pores with cross-sectional diameters of from about 2 nanometers to about 50 nanometers. Macroporous materials include those having pores with cross-sectional diameters of greater than about 50 nanometers and as large as 1 micrometer.

As used herein, the term "peptide" has its ordinary meaning in the art and may refer to amides derived from two or more amino carboxylic acid molecules (the same or different) by formation of a covalent bond from the carbonyl carbon of one to the nitrogen atom of another with formal loss of water. An "amino acid residue" also has its ordinary meaning in the art and refers to the composition of an amino acid (either as a single amino acid or as part of a peptide) after it has combined with a peptide, another amino acid, or an amino acid residue. Generally, when an amino acid combines with another amino acid or amino acid residue, water is removed, and what remains of the amino acid is called an amino acid residue. The term "amino acid" also has its ordinary meaning in the art and may include proteogenic and non-proteogenic amino acids. In some embodiments, an amino acid may be in carboxylate form. In some embodiments, an amino acid may be carboxylic acid form.

As used herein, the term "protecting group" is given its ordinary meaning in the art. Protecting groups include chemical moieties that are attached to or are configured to be attached to reactive groups (i.e., the protected groups) within a molecule (e.g., peptides) such that the protecting groups prevent or otherwise inhibit the protected groups from reacting. Protection may occur by attaching the protecting group to the molecule. Deprotection may occur when the protecting group is removed from the molecule, for example, by a chemical transformation which removes the protecting group.

The following examples are intended to illustrate certain embodiments of the present invention, but do not exemplify the full scope of the invention.

## EXAMPLE 1

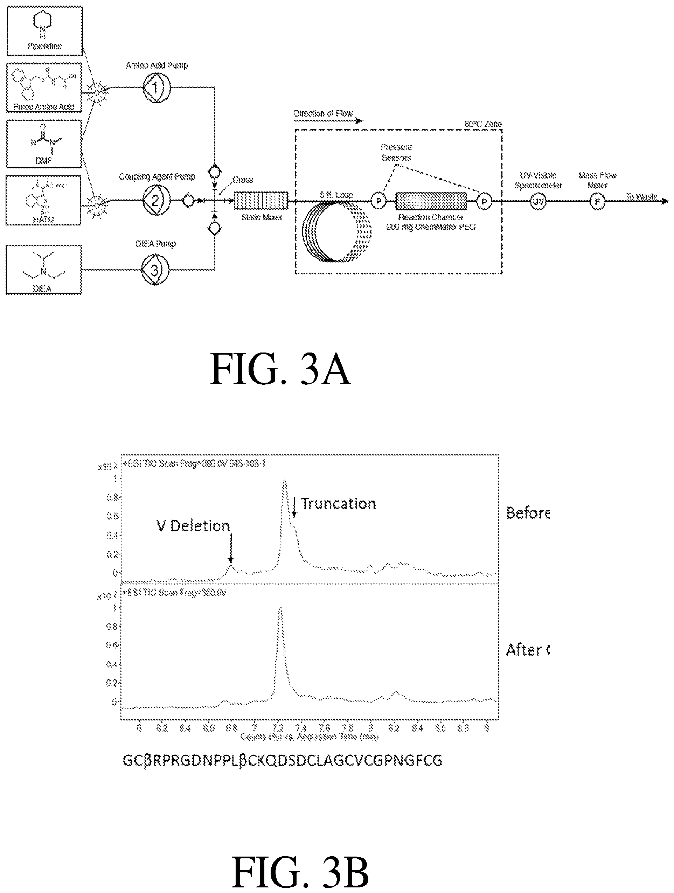

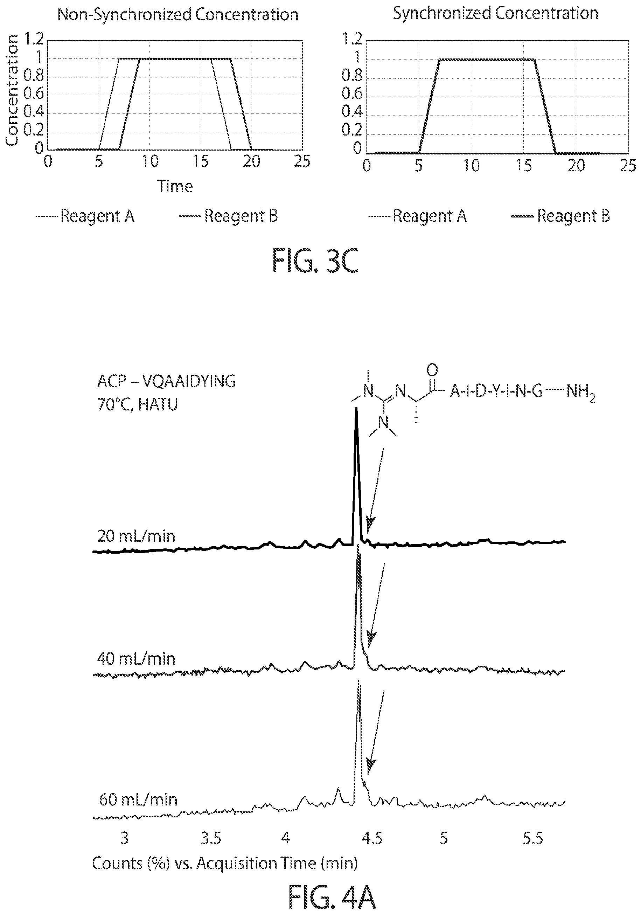

The device shown in FIG.3 comprises three pumps connected downstream to a fluidic manifold where the fluid streams merge at a point. One of the three pumps was connected upstream to a manifold for selection of an amino acid from one of many reservoirs. The second pump is connected upstream to a manifold for selection of an activating agent from one of many reservoirs. The third pump was connected to a reservoir containing a base or a manifold for selection of a base from one of many reservoirs.

To perform the coupling steps as described in the subsequent examples, the valves were switched to select the desired activating agent, amino acid, and base. Then, the pumps are activated such that the leading edges of the three fluid streams meet simultaneously at the merging point. This pumping cycle was continued until the operator or software desires to terminate the coupling, at which point the valves were changed to a wash solvent. The pumps were then activated such that the trailing edges of the fluid streams are matched.

## EXAMPLE 2

This example describes peptide synthesis using the merging techniques described in Example 1 and peptide synthesis using conventional methods.

Briefly, EETI-II, sequence shown in FIG. 3B, was synthesized using the activation method of Example 1. As a control, the same peptide was synthesized using method wherein the fronts of the fluid streams were not merged simultaneously. Both peptides were synthesized using standard synthesis parameters with HBTU at 70°C and 20 mL/min total flow rate.

In the control synthesis, the ratio of activation reagents in the fluid streams were at the desired ratio(s), because substantially simultaneous merging did not occur, and significant truncation was observed in the LC-MS trace of the crude peptide as shown in FIG. 3B (before). The truncation corresponded to a slug of unreacted activating agent being introduced into the reactor. As shown in FIG. 3C, if an activation reagent arrives at the mixing region before another activation reagent, the molar ratio of the activation reagent will vary in the fluid stream. After the concentration profiles of the fluid streams were matched using the methods described in Example 1, the crude peptide did not have the truncation and was much more easily purified as shown in FIG. 3B (after).

## EXAMPLE 3 (reference)

This example describes the effect of pump timing on the substantially simultaneous merging of fluid streams to activate amino acids. Mismatch in pump timing resulted in side reactions.

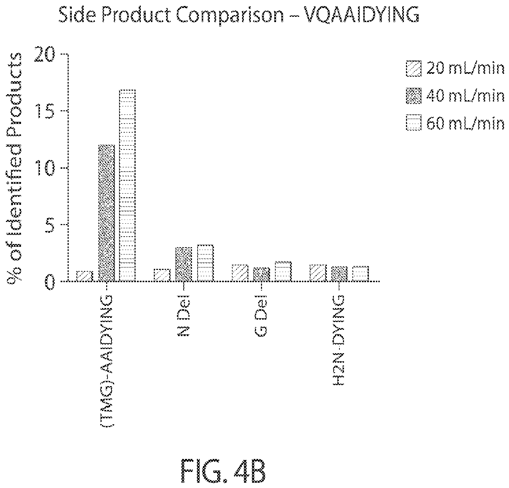

Briefly, the peptide ACP was synthesized at varying flow rates using a solid phase peptide synthesizer that had a mismatch in pump timing, such that the activation reagents did not arrive at the junction as the same time. Because of the mismatch in pump timing, differences in reagent flow become more exaggerated at elevated flow rates. This was exemplified by the presence of a truncation product, (TMG)-AAIDYING, indicated by the arrows in FIG. 4A. Truncation was a result of coupling between unmixed activating agent and an immobilized peptide. As the flow rate increased, the proportion of the truncation product in the resulting LC-MS chromatogram increased, while deletion side products remain relatively constant.

## EXAMPLE 4

This example describes peptide synthesis using the merging techniques described in Example 1.

Amide bond forming reactions are prevalent in the syntheses of therapeutic small molecules, peptides, and proteins. Of 128 recently surveyed small molecule drug candidates, 65% required formation of an amide. In addition to small molecules, peptides, including GLP-1 agonists for diabetes treatment, require forming up to 40 amide bonds. Personalized peptide vaccines, a frontier in cancer treatment, require custom synthesis for each patient. However, research, development, and production of these peptides is limited by synthesis speed, typically minutes to hours for each amino acid addition and deprotection cycle. In this example, we report a fully automated, flow chemistry approach to solid phase polypeptide synthesis with amide bond formation in seven seconds and complete cycle times in forty seconds is described. Crude peptide qualities and isolated yields were comparable to standard batch solid phase peptide synthesis. At full capacity, this machine could synthesize 25,000 30-mer individual peptides per year weighing a combined 25 kilograms.

Peptides and proteins are important in the search for new therapeutics. Underpinning peptide and protein research is the need to design new functional variants and to quickly iterate on these designs. Biological expression of peptides can be fast and scalable - the ribosome synthesizes peptides at a rate of 15 peptide bonds per second - but becomes difficult outside of the twenty, naturally-occurring amino acids. On the other hand, despite the expanded number of monomers, chemical peptide synthesis remains relatively slow. In this example, Automated Flow Peptide Synthesis (AFPS), a method with the flexibility of chemical synthesis that approaches the speed of the ribosome is described. AFPS reduces the amide bond forming step to seven seconds and the entire cycle for each amino acid addition to 40 seconds while maintaining a high level of control over the chemistry. UV monitoring and disposable reactors allow for yield quantitation and fast, automated switchover.

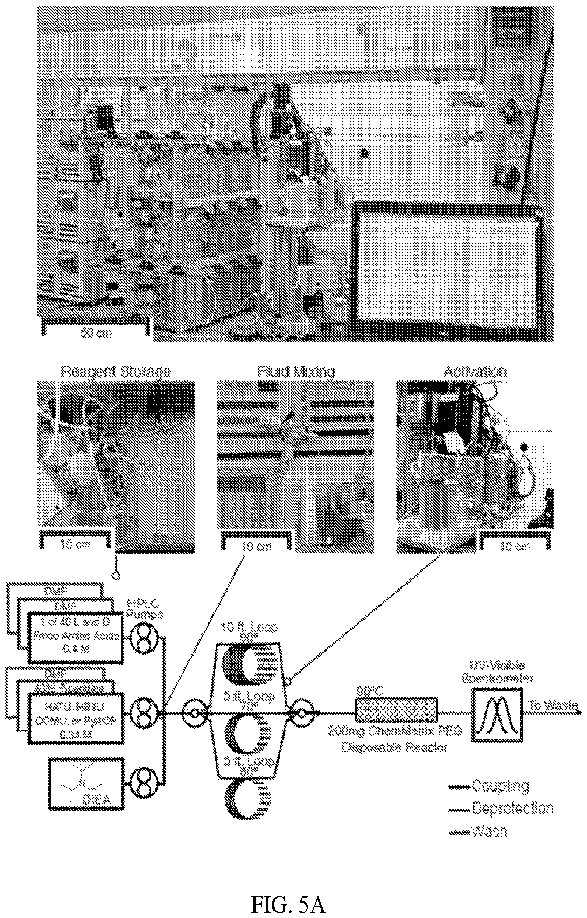

The Automated Flow Peptide Synthesizer consists of five modules, depicted in FIGs. 5A-5B. During a coupling reaction, the machine draws reagents from the storage module, and then mixes the desired amino acid with an amine base (diisopropylethylamine, DIEA), and an activating agent (e.g. HATU or PyAOP) in the mixing module. This mixture flows through the activation module, an electrically heated plug flow reactor, where it quickly heats to 90°C. Within two seconds of activation, the activated amino acid flows through the coupling module, a packed bed of peptide synthesis resin, where amide bond formation is complete within seven seconds. The resin is contained in a 6-mL disposable syringe cartridge for easy removal. The AFPS monitors Fmoc removal for each cycle by recording the absorbance of the reactor effluent as a function of time. The Fmoc removal absorbance chromatogram allows the deprotection efficiency, the coupling yield, and the rate of material flux through the peptidyl resin to be inferred, which allowed for the identification of on-resin peptide aggregation.

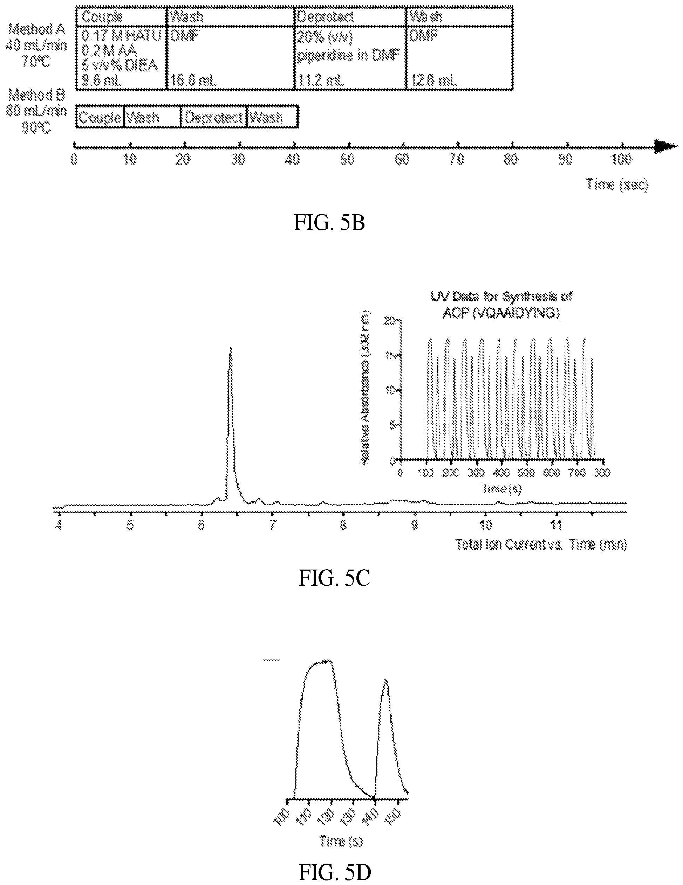

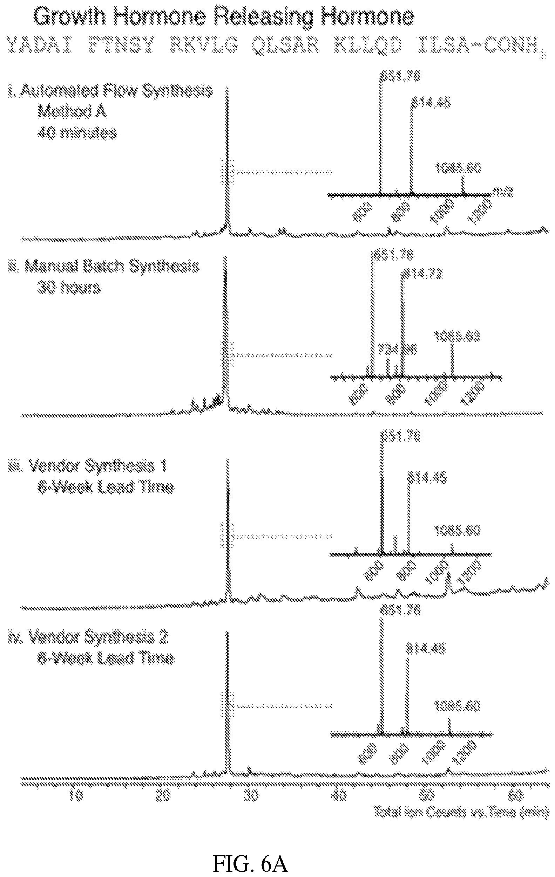

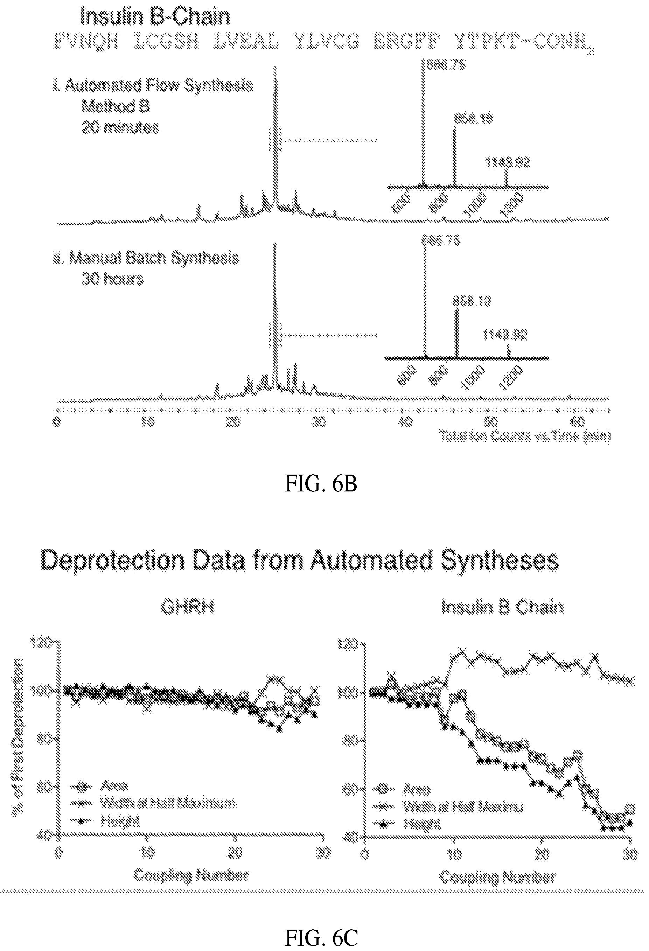

The AFPS was initially validated by synthesizing test peptides ALFALFA and a fragment of acyl carrier protein ($ACP_{65-74}$) as shown in FIG. 5D. These peptides were synthesized in high yield with low levels of side products. A comparative study was then performed between longer peptides produced by the AFPS, batch synthesis, and reputable custom peptide vendors, as shown in FIGs. 6A-6B. Compared to standard batch methods, peptide synthesis using high-speed continuous flow activation at elevated temperatures allowed for comparable or higher quality synthesis of long polypeptides in a fraction of the time. Additionally, as shown in FIG. 6C, in-process UV monitoring gave information about the synthetic yields of each step. The steady decrease in peak area observed for the insulin B chain resulted from chain-terminating side reactions. These byproducts appeared as a series of impurities around the main peak in the LC-MS chromatogram.

The epimerization of Cys and His with high-temperature flow activation was then assessed. When activated, Cys and His can lose stereochemistry at the $C^\alpha$

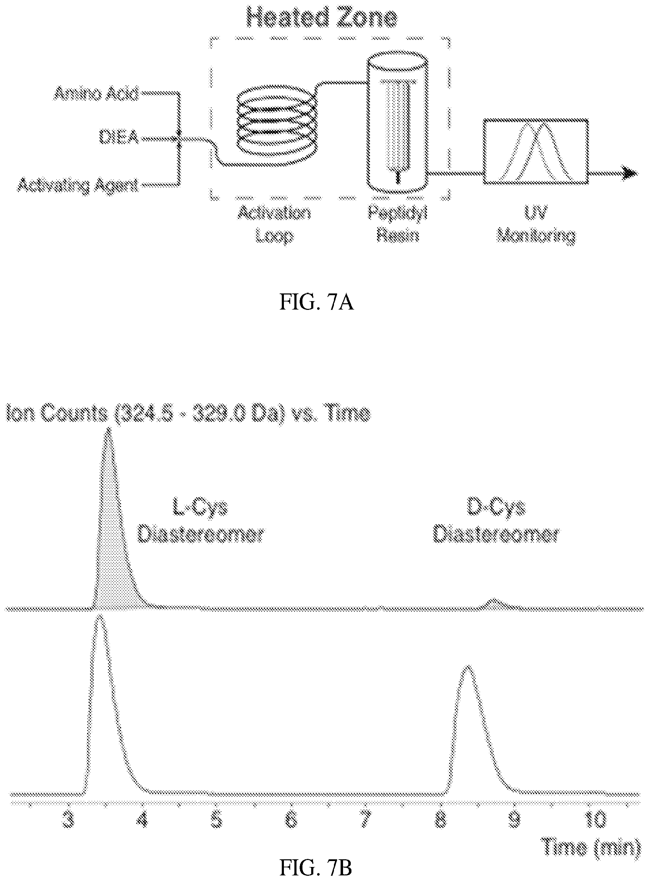

position. This problem bedevils batch synthesis techniques, especially at elevated temperature, because activation, coupling, and degradation all happen simultaneously in the same vessel. On the batch microwave synthesizer, if has been found coupling Fmoc-L-Cys(Trt) for 1.5 minutes at 90°C under microwave irradiation with HBTU and DIEA causes 16.7% of the undesired D-Cys product to form. In contrast, it was found that continuous flow allows the activation process to be controlled by the amount of time in the heated zone of the system shown in FIG. 7A. To probe this, two model peptides FHL and GCF, whose diastereomers can be separated and quantified by LC-MS were used. By increasing the flow rate, and therefore decreasing the residence time at temperature of activated Fmoc-Cys(Trt) and Fmoc-His(Boc), the diastereomer formation was limited for AFPS method B to 0.5% for FHL and 3% for GCF. This level of diastereomer formation is consistent with optimized room temperature batch synthesis protocols.

The method described in this example offers numerous advantages over manual flow synthesis, thermally-accelerated batch synthesis, and other continuous flow peptide synthesis methods. First, automation of the entire process of heating, mixing, and activation of amino acids in a mix-and-match format enables endless possibilities to tune chemistry on a residue-by-residue basis. Second, inline mixing of these reagents with precise pump and valve actuation allows for control of stoichiometry, residence time, and amino acid epimerization, making the synthesis highly reproducible.

FIG. 5A shows a photograph of the automated flow solid phase synthesizer, highlighting the different system modules and a process flow diagram. Amino acid, activating agent and DIEA are merged together by three HPLC pumps. A series of multiposition valves controls the selection of the amino acid and activating agent. Amino acid activation occurs by flow through one of several heated flow paths determined by the position of a column selector valve. Activated amino acid is then flowed over a resin bed containing 200 mg of peptidyl resin housed in a 6-mL fritted polypropylene syringe that is sheathed by a heated jacket. The waste effluent is passed through a UV-visible spectrometer and then to waste. FIG. 5B shows a cycle diagrams showing the duration of each step, the solution composition during each step after mixing, and the total volume of reagent used at each step. FIG. 5C shows LC-MS data for the crude product of acyl carrier protein (65-74) synthesis using Method B, synthesized in 44% isolated yield. For this synthesis, 200 mg of starting peptidyl resin yielded 314 mg of dried resin. Throughout this work, isolated crude peptide yields are based on the nominal loading of resin. FIG. 5D shows an example of UV absorbance data for one coupling and deprotection cycle.

FIG. 6A shows LC-MS data Growth Hormone Releasing Hormone synthesized via different methods. Growth hormone releasing hormone was synthesized in (i) 40 minutes with method A in 58% isolated yield, compared to (ii) 30 hours using manual batch techniques with a 60% isolated yield. This peptide was also purchased from two vendors (iii, iv) with a 6-week lead time. Cleavage of 200 mg of each of these peptidyl resins yielded 76 mg and 90 mg, amounts comparable to automated and manual syntheses. FIG. 6B shows LC-MS data for Insulin B-chain synthesized using different methods. The insulin B-chain was synthesized in 20 minutes using Method B in 53% isolated yield, compared to 30 hours and in 45% yield with manual batch techniques. FIG. 6C shows a plot of Fmoc deprotection UV data for each cycle of synthesis for GHRH and Insulin B-chain. Peak area, full-width half maximum, and peak maximum is plotted as a function of coupling number. Liquid chromatography and ESI-MS was performed on an Agilent 1260 Infinity LC tethered to a 6520 QTOF mass spectrometer. Each sample was injected onto a Zorbax 300SB-C3 column pre-equilibrated with 5% acetonitrile in water with 0.1% formic acid. After a 4 minute hold, the acetonitrile concentration was ramped to 65% over 60 minutes.

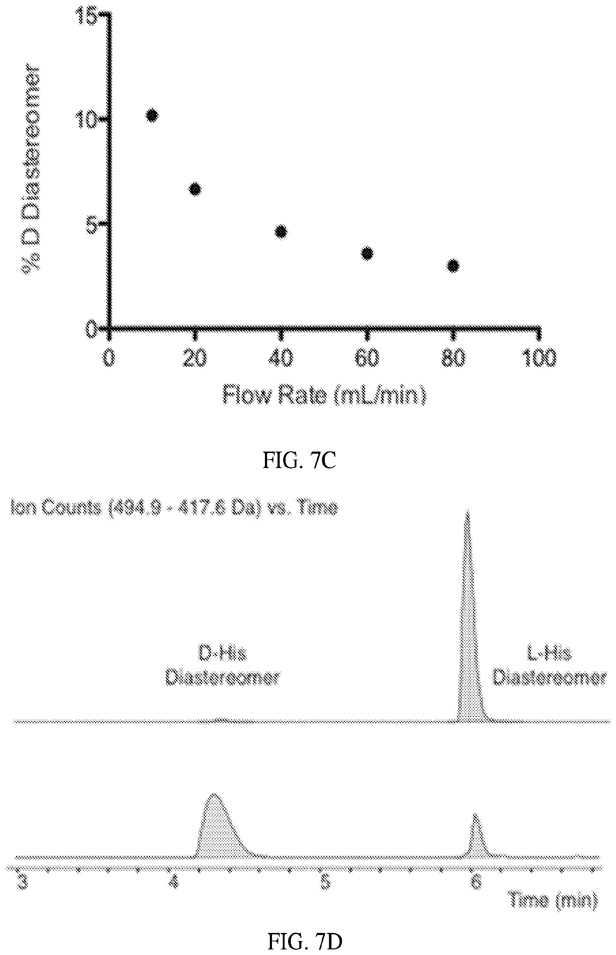

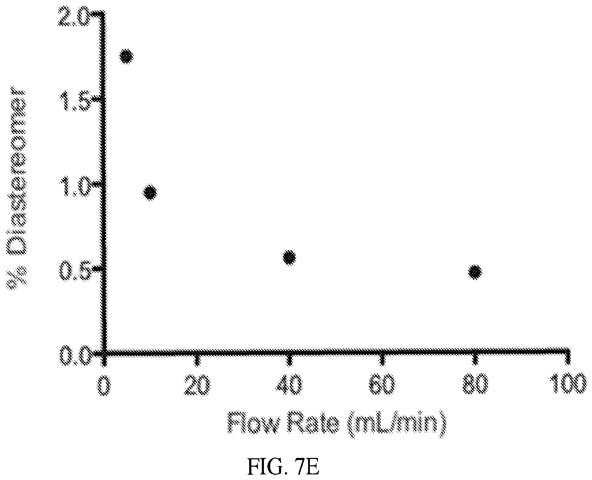

FIG. 7A shows a diagram of the heated portion of the automated flow peptide synthesizer. FIG. 7B shows a diastereomer analysis of model peptide GCF showing a representative sample from flow synthesis using method B (top) and a 50/50 mixture of the authentic Cys diastereomers (bottom). FIG. 7C shows the percentage of Cys diastereomer formation as a function of flow rate (ml/min) using method B. FIG. 7D shows the same analysis as FIG. 7B for model peptide FHL to investigate His epimerization during flow activation. LC-MS of model peptide FHL synthesized using method B (top panel) and a 50/50 mixture of authentic His diastereomers (bottom). FIG. 7E shows the percentage of histidine diastereomer formation as a function of flow rate (ml/min).

## EXAMPLE 5

This example describes the materials, methods, and instrument configuration used in Example 4.

Materials: All reagents were purchased and used as received. Fmoc amino acids were purchased from Creo Salus. Fmoc-His(Boc)-OH and O-(7-Azabenzotriazol-1-yl)-N,N,N',N'-tetramethyluronium hexafluorophosphate (HATU) was purchased from ChemImpex. Omnisolv grade N,N-dimethylformamide (DMF) was purchased from EMD Millipore (DX1726-1). Diisopropylethylamine (DIEA, catalog number 387649), piperidine, trifluoroacetic acid, triisopropylsilane, acetonitrile and 1,2-ethanedithiol (EDT) were purchased from Sigma Aldrich. H-Rink Amide ChemMatrix polyethylene glycol resin was purchased from Pcas Biomatrix (catalog number 1744).

Reagent Storage and Fluidic Manifold: The reagent storage system used two different vessels to contain reagents: a Chemglass three-neck 500 mL spinner flask for large volumes (CLS-1401-500), and 50 mL polypropylene syringe tubes for smaller volumes (parts # AD930-N, AD955-N). All of the glass bottles were painted with a UV-resistant matte spray paint (Krylon 1309) to reduce UV degradation of the reagents and had a green protective safety net for operation under argon pressure. The argon pressure was maintained at 5 psi pressure with a Swagelok pressure regulator (part# KCP1CFB2B7P60000). The reagent withdraw lines were outfitted with a 20 um polypropylene filter (part# JR-32178) to prevent clogging of pumps, check valves, and lines from any reagent crystallization or impurities.

Each row of 9 amino acid bottles and syringes fed into a VICI Valco 10-position valve (Vici part# C25-3180EUHA) where the tenth position was DMF. Those valves all fed into a main Vici Valco 10 position valve. This main valve fed the amino acid pump. Bottles containing HATU, other coupling agents, 40% piperidine, and DMF fed into a separate 10-position valve. This valve was connected to the coupling agent pump. DIEA feeds directly into the third pump.

Pumping and Mixing: The AFPS operated with three Varian Prostar 210 pumps. The first pump delivered either an amino acid or DMF. The second pump delivered either a coupling agent, 40% piperidine solution, or DMF. The third pump delivered DIEA. The coupling agent and amino acid pumps had a 50 ml/min stainless steel pump head (Agilent part# 50-WSS). The DIEA pump had a 5 ml/min pump head (Agilent part# 5-Ti). The three pumps outlets merged at a cross (IDEX part# P-722) with three inlet check valves (IDEX part# CV-3320) to prevent diffusion between the cross and pump head. The lengths of PEEK tubing (1/16" OD, 0.020" ID) between the PEEK cross and all of the pumps had matched volumes. After the cross, a length FEP tubing (1/16" OD, 0.030"ID) was coiled 22 times around a ½ inch cylinder to form a high dean number (> 3000) static mixer to facilitate reagent mixing.

Activation and Coupling Reactors: After mixing, the reagent stream proceeded to a heat exchanger that was selected using a VICI Valco six-position column selector valve (Vici part# ACST6UW-EUTA). These heat exchangers consisted of a length of stainless steel tubing wrapped around an aluminum spool and coated with silicone for insulation. The spools were heated with two resistive cartridge heaters (Omega part# CSS-10250/120V). For peptide synthesis method A, a 3m (10 ft, 1.368 ml) heat exchanger loop at 90°C was used; for peptide synthesis method B, a 1.5m (5 ft, 0.684 ml) heat exchanger loop at 70°C was used.

Prototyping on Arduino: Initially, the control system was prototyped on an Arduino Mega. The pumps and valves were daisy chained and connected to separate TTL serial ports on the Arduino using the RS232 MAX3232 SparkFun Transceiver Breakout (BOB-11189)

Serial Communication with Pumps and Valves: Standard RS-485 serial protocols were used for communication with the Varian ProStar 210 pumps and VICI Valco valves. Pump communication was at 19200 baud, 8 bit, even parity, with 1 stop bit. Valve communication was at 9600 baud with no parity and one stop bit.

Heating and Temperature Control: All heaters were controlled with an 8-channel Watlow EZ-Zone RM controller (part number RMHF-1122-A1AA). This controller integrates PID control on-board. Temperatures were read into the software through the RS-232 serial port using software provided by Watlow. All thermocouples were calibrated using a single point calibration at 0 degrees Celsius.

Process Data Collection: The software recorded temperature, mass flow rate, pressure, and UV absorbance during each synthesis. The Watlow PID control unit described above was used to acquire temperature data. For mass flow data, a Bronkhorst Coriolis mass flow meter was used (part# M14-XAD-11-0-5) and also allowed monitoring of fluid density. The differential pressure across the reactor was monitored using two DJ instrument HPLC through-bore titanium pressure sensors (part# DF2-01-TI-500-5V-41"). These sensors were single point calibrated at 90 degrees Celsius at 100 psi.

UV monitoring at 312 nm was accomplished by using a Varian Prostar 230 UV-Vis detector fitted with a super prep dual path length flow cell (nominal path lengths of 4 mm and 0.15 mm). This dual path length flow cell setup allowed for high dynamic range absorbance measurements - whenever the absorbance increased past the linear range for the large flow cell, the instrument switched to recording the absorbance through the smaller flow cell. In order to assure accurate measurements during the flow cell switchover, the ratio of path lengths was calibrated using a standard solution of dibenzofulvene prepared as described in Letters in Peptide Science, 9: 203-206, 2002.

Temperature and mass flow data were acquired through serial communication with the Watlow PID and Bronkhorst flow meter. Electronic voltage measurements for pressure and UV data were obtained from the instrument using a National Instruments NI cDAQ-9184 (part number 782069-01) with a NI 9205 32-channel analog input card (part number 779357-01). Data points were recorded with averaging every 50 ms. On the UV detector, the signal response time was set to 10 ms and the full voltage scale was 100 mv.

The software allowed for customization of amino acid, activating agent, temperatures of the coupling and deprotection steps, flow rate of the coupling and deprotection steps, and the amount of reagents used (number of pump strokes). These could be modified while the system was in operation: for instance, in response to UV that suggested aggregation, the temperature, the amount of amino acid used, or the activating agent could be changed.

The synthesizer was controlled over Ethernet and USB on a Windows computer with a LabView VI. The VI has a graphical interface to allow a user to easily create a recipe for the desired peptide. Recipes allow users to control the flow rate, the amount of amino acid used, the activating agent, the temperature and residence time of activation, the deprotection residence time, and the amount of deprotection reagent for each step of the synthesis. Once, the user has created the desired recipe, he or she submits it to the machine queue and presses "Run." During the synthesis, the recipe can be modified for any subsequent coupling step on the fly. When "Run" is pressed, the software populates the predefined routine for each amino acid with the users selected amino acid, flow rates, temperatures, amount of reagents, and type of activating reagent.

The code consisted of operations performed on either pumps, valves, or motors. Each operation consisted of a set of inputs and a dwell time. Valves accept a valve ID and valve position; pumps accept a pump ID and pump flow rate; motors accept a motor ID and motor position. After a step was complete, the program waited until completion of the dwell time before executing the next step. Dwell times represented by #variables are computed on the fly using the recipe input. For instance, the dwell time after actuation of the pumps in step 12 is determined by the "CPL NStrk" (number of coupling strokes) parameter in the recipe.

Analytical Peptide Cleavage and Side Chain Protecting Group Removal: Approximately 10 mg of peptidyl resin was added to a 1.5 mL Eppendorf tube. 200 μL of cleavage solution (94% TFA, 1% TIPS, 2.5% EDT, 2.5% water) was added to the tube and incubated at 60°C for 5 minutes. After completion of cleavage, 200μL TFA was added to the tube to rinse the resin, and as much liquid as possible was transferred into another tube using a pipet tip, avoiding resin. To the tube of cleavage solution, 800μL cold diethyl ether was added. The tube was shaken - a visible waxy precipitate formed and was collected by centrifugation. The supernatant ether was poured off and two more ether washes were performed.

Finally, the waxy solid was allowed to dry briefly under a stream of nitrogen gas. 500μL of 50% acetonitrile in water was added to the tube and mixed thoroughly. This solution was filtered through a centrifugal basket filter and diluted 1:10 in 50% acetonitrile in water with 0.1% TFA for the liquid chromatographic analysis.

Preparative Peptide Cleavage: After synthesis, peptidyl resin was washed with dichloromethane, dried in a vacuum chamber, and weighed. The resin was transferred into a 15 mL conical polypropylene tube. Approximately 7 mL of cleavage solution (94% TFA, 1% TIPS, 2.5% EDT, 2.5% water) was added to the tube. More cleavage solution was added to ensure complete submersion. The tube was capped, inverted to mix every half hour, and was allowed to proceed at room temperature for 2 hours.

Then, the resin slurry was filtered through a 10 μm polyethylene membrane disk fitted into a 10 mL Torviq syringe. The resin was rinsed twice more with 1 mL TFA, and the filtrate was transferred into a 50 mL polypropylene conical tube. 35 mL ice cold diethyl ether were added to the filtrate and left to stand for 30 minutes to precipitate the peptide. The precipitate was collected by centrifugation and triturated twice more with 35 mL cold diethyl ether. The supernatant was discarded.

Finally, residual ether was allowed to evaporate and the peptide was dissolved in 50% acetonitrile in water. The peptide solution was frozen, lyophilized until dry, and weighed.

Analytical Liquid Chromatographic Analysis of Peptide Samples: 1μL of the diluted peptide sample was analyzed on an Agilent 6520 LC-MS with a Zorbax 300SB-C3 column (2.1 mm x 150 mm, 5μm particle size). For samples in FIGs. 6A-6C, a gradient of acetonitrile in water with a 0.1% formic acid additive was used. Gradients started at 5% acetonitrile and ramped to 65% acetonitrile at a rate of 1% acetonitrile per minute. The full method included a hold time at 1% along with total time of gradient

Initial Synthesis Conditions and System Characterization: At 20 mL•min-1 total system flow rate and at 70°C, treatment with 20% piperidine was chosen to be 20s, conditions that were previously shown to be sufficient for complete Fmoc removal. The DMF washes were chosen to be 30s. The washout time was verified by introducing Fmoc amino acid into the reactor and using the UV detector to ensure that the system was cleared of any UV active material after the DMF wash.

The scheme for in-line mixing the fluid streams of activating agent and the amino acid allowed for versatility in the conditions used for coupling. However, it required a departure from the conditions traditionally used for aminoacylation in Fmoc synthesis. Typically, reagents are used at their solubility limits, around 0.4M for Fmoc amino acids and uronium coupling agents. However, because these reagents were stored separately on the AFPS and coupling involved mixing two concentrated solutions, the final solution used for aminoacylation at the outset was composed of 0.2M amino acid and activating agent. For the typical coupling, a total of 9.6 mL of this coupling solution was used to ensure complete coupling. These conditions were initially tested for the synthesis of a short polypeptide, ALFALFA.

Optimization of Synthesis Cycle: A 10-residue peptide that is typically used as a diagnostic "difficult" sequence, ACP, was synthesized at 70°C, using the same volume of coupling reagent in each experiment, at 20, 40, and 60 mL/min total flow rate. At higher flow rates, the increasing formation of a chain termination side product - a tetramethylguanidyl truncation during the glutamine coupling was observed. It was hypothesized that this was due to incomplete activation at elevated flow rates: when the amounts of activating agent and amino acid are nearly equal, there could be residual HATU present which can guanidinylate the N-terminus of the growing peptidyl chain. Reducing the concentration of activating agent to 0.34M, as well as ensuring full synchronization of the pump heads eliminated this side reaction in most cases, allowing us to synthesize ACP at 80 mL/min in quantitative yield. For Fmoc-Arg couplings in other peptides, these truncations were still observed, so PyAOP was used as the activating agent for these couplings.

Investigation of Temperature Effect on Deprotection: The deprotection of Fmoc-Glycine-functionalized peptidyl resin with 20% piperidine at 70, 80, and 90°C was examined. In all three cases, Fmoc-Gly was coupled to 200 mg of ChemMatrix Rink Amide resin at room temperature using batch coupling methods. The resins were then transferred to the automated flow synthesizer, where a single treatment of 20% piperidine was performed at either 70, 80, or 90°C. In all three cases, the integrated area of the Fmoc removal peaks was the same, suggesting complete Fmoc removal. However, at higher temperatures, the peak maximum occurs earlier, suggesting either faster deprotection, faster diffusion of the Fmoc-dibenzofulvene adduct out of the resin, or both.

Representative Protocol for Synthesis of Peptides on the Automated Flow Peptide Synthesizer: 200 mg of ChemMatrix PEG Rink Amide resin was loaded into a 6 mL Torviq fritted syringe fitted with an additional 7-12 $\mu$m Porex UHMWPE (XS-POR-7474) membrane on top of the frit. The resin was preswollen with DMF for 5 minutes, after which large resin aggregates were manually broken up by inserting the syringe plunger. The syringe was filled with DMF, loaded onto the fluidic inlet, and loaded into a 90°C heated chamber. The synthesizer was set up as shown in FIG. 5A, with all reagents pumped at a total flow rate of 80 mL·min-1 though a cross manifold, a mixer, and a 10ft stainless steel heated loop at 90°C before being pumped over the resin. Three Varian Prostar 210 HPLC pumps were used, two with 50 mL·min-1 pump heads for amino acid and activating agent, and one with a 5 mL·min-1 pump head, for diisopropylethylamine (DIEA). The 50 mL·min-1 pump head pumped 400 $\mu$L of liquid per pump stroke; the 5 mL·min-1 pump head pumped 40 $\mu$L of liquid per pump stroke.

The standard synthetic cycle used involved a first step of prewashing the resin at elevated temperatures for 20s at 80 mL/min. During the coupling step, three HPLC pumps were used: a 50 mL·min-1 pump head pumped the activating agent (typically 0.34 M HATU), a second 50 mL·min-1 pump head pumped the amino acid (0.4M) and a 5 mL·min-1 pump head pumped diisopropylethylamine (DIEA). The first two pumps were activated for 5 pumping strokes in order to prime the coupling agent and amino acid before the DIEA pump was activated. The three pumps were then actuated together for a period of 7 pumping strokes, after which the activating agent pump and amino acid pump were switched using a rotary valve to select DMF. The three pumps were actuated together for a final 5 pumping strokes, after which the DIEA pump was shut off and the other two pumps continue to wash the resin for another 16 pump strokes.

During the deprotection step, two HPLC pumps were used. Using a rotary valve, one HPLC pump selects 40% piperidine and the other selects DMF. The pumps were activated for 13 pump strokes. After mixing, the final concentration of piperidine is 20%. Next, the rotary valves select DMF for both HPLC pumps, and the resin was washed for an additional 16 pump strokes. The coupling/deprotection cycle was repeated for all additional monomers.

Aspartimide Formation and Elevated Temperature GHRH Synthesis: GHRH synthesis at 70°C and at 90°C was investigated. When performing this synthesis at 90°C, as opposed to 70°C, formation of an aspartimide byproduct with a signature -18 Da mass and shifted retention time was noticed. This side reaction is known to happen both at elevated temperature and with particular Asp-containing peptides. The effect of piperazine, a milder base, on this side reaction was investigated. Use of 2.5% piperazine instead of 20% piperidine for the deprotection significantly reduced the amount of this side product as measured by LC-MS, but increased the amount of amino acid deletions, particularly Ala and Leu. Addition of 0.1 M HOBt to the 2.5% piperazine deprotection cocktail resulted in roughly the same synthesis quality. For Asp-containing peptides where aspartimide formation is suspected, it is therefore advantageous to use either reduced temperature, a reduced strength deprotection cocktail, or both.

Manual Synthesis of Insulin B chain and GHRH: These peptides were synthesized according to Kent, et al., Org Lett. 2015, 17 (14), 3521. ChemMatrix Rinkamide resin (0.1 mmol; 0.45 mmol/g) was used. Amino acids were activated for 30 seconds by first dissolving 0.55 mmol of the amino acid to be coupled in 1.25 mL 0.4 M HBTU/0.4 M HOBT, and then adding 122 $\mu$L (0.7 mmol) of DIEA. After 30 seconds, the solution was added to the resin. The couplings were allowed to proceed for 30 minutes with intermittent stirring.

After each coupling step, a 45 mL DMF flow wash was performed. Then, 3 mL of 20% (v/v) piperidine was added to the resin, stirred, and allowed to incubate for 5 minutes. This process was repeated once. After each deprotection, a 45 mL flow wash was performed, followed by a 1 minute batch treatment with DMF.

Determination of Cys and His Epimerization: Cys and His epimerization were measured using the two model peptides GCF and FHL, respectively. For each synthesis, the flow rates for C and H coupling were varied, and the coupling conditions for the flanking residues (G and F for GCF; F and L for FHL) were kept constant at 90°C and 80 mL/min total flow rate. After synthesis of each model peptide, cleavage was performed as described above.

LC-MS analysis of the cleaved product was performed. In order to determine the amount of D-epimer formed in each case, extracted ion chromatograms of the two stereoisomers were obtained: 342.5-329.0 Da for GCF and 494.9-417.6 Da for FHL. The peaks corresponding to each epimer were integrated. Authentic standards were prepared and analyzed on the same methods in order to verify the retention times of each epimer.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," and "holding," are to be understood to be open-ended, i.e., to mean including but not limited to. Only the transitional phrases "consisting of" and "consisting essentially of' shall be closed or semi-closed transitional phrases, respectively, as set forth in the United States Patent Office Manual of Patent Examining Procedures, Section 2111.03.

## Claims

1. A method of initiating operation of a peptide synthesis system, comprising:

   commencing flow of a first fluid stream comprising amino acids from a first reagent reservoir to a mixing region;
   commencing flow of a second fluid stream comprising a base from a second reagent reservoir to the mixing region, such that a leading edge of the first fluid stream and a leading edge of the second fluid stream arrive at the mixing region within about 10 ms of each other;
   optionally commencing flow of a third fluid stream comprising an activating agent;
   merging the first and second fluid streams, and optionally the third fluid stream, at a mixing region to form a mixed fluid stream; and
   flowing the mixed fluid stream to a reactor.

2. The method of claim 1, comprising commencing the flow of the third fluid stream comprising the activating agent and merging the first, second, and third fluid streams at the mixing region to form the mixed fluid stream.

3. A method of initiating operation of a peptide synthesis system, comprising:

   commencing flow of a first fluid stream comprising activating agent from a first reagent reservoir to a mixing region;
   commencing flow of a second fluid stream comprising a base from a second reagent reservoir to the mixing region, such that a leading edge of the first fluid stream and a leading edge of the second fluid stream arrive at the mixing region within about 10 ms of each other;
   optionally commencing flow of a third fluid stream comprising amino acids;
   merging the first and second fluid streams, and optionally the third fluid stream, at a mixing region to form a mixed fluid stream; and

flowing the mixed fluid stream to a reactor.

4.  The method of claim 3, comprising commencing the flow of the third fluid stream comprising the amino acids and merging the first, second, and third fluid streams at the mixing region to form the mixed fluid stream.

5.  The method of any preceding claim, comprising flowing a fourth fluid stream comprising an additive selected from the group consisting of a chaotropic salt, a cosolvent, and a surfactant, and further comprising merging the first, second, third and fourth fluid streams at the mixing region.

6.  The method of any preceding claim, wherein:

    a) a molar ratio of the amino acids to the base measured at the leading edge as the leading edge enters the reactor is within 10% of a molar ratio of the base to the activating agent in the mixed fluid stream at the mixing region; and/or
    b) a molar ratio of the base to the activating agent measured at the leading edge as the leading edge enters the reactor is within 10% of a molar ratio of the base to the activating agent in the mixed fluid stream at the mixing region; and/or
    c) a molar ratio of the amino acids to the base measured at the leading edge as the leading edge enters the reactor is within 10% of a molar ratio of the activating agent to the base in the mixed fluid stream at the entrance to the reactor at a time that is at least about 10 ms after the leading edge enters the reactor; and/or
    d) a molar ratio of the base to the activating agent measured at the leading edge as the leading edge enters the reactor is within 10% of a molar ratio of the activating agent to the base in the mixed fluid stream at the entrance to the reactor at a time that is at least about 10 ms after the leading edge enters the reactor.

7.  The method of any preceding claim, wherein a molar ratio of amino acids to base in the mixed fluid stream is more than about 1:1.

8.  The method of any preceding claim, wherein the activating agent is selected from the group consisting of a carbodiimide, guanidinium salt, phosphonium salt, and uronium salt.

9.  The method of any preceding claim, wherein:

    a) the base is a Lewis base; and/or
    b) the base is a non-nucleophilic base.

10. The method of any preceding claim, wherein:

    a) the mixed fluid stream is not exposed to heat from a heat source prior to arrival at reactor; and/or
    b) the mixed fluid stream is not exposed to heat from a heat source between the mixing region and the entrance to the reactor.

11. The method of any preceding claim, wherein:

    a) the temperature of the mixed fluid stream is within 10°C of the temperature of the leading edge at the entrance of the reactor; and/or
    b) the temperature of the leading edge varies by less than 10°C from the mixing region to the entrance of the reactor.

12. The method of any preceding claim, wherein the reactor does not comprise a plurality of amino acids and/or a plurality of peptides immobilized on a solid support.

13. The method of any of claims 1-12, wherein:
    the reactor comprises a plurality of peptides immobilized on a solid support, optionally wherein:

    a) the solid support is contained within a packed column and/or a fluidized bed, and/or the solid support comprises a resin, a microporous polystyrene resin, a microporous polyethylene glycol resin, and/or a microporous co-polymer resin; and/or
    b) the method further comprises exposing the amino acids to the immobilized peptides such that at least a portion of the amino acids are bonded to the immobilized peptides to form elongated peptides; and/or
    c) greater than about 99% of the immobilized peptides each become bonded to a single amino acid molecule during the exposing step.

14. The method of any preceding claim:
    further comprising providing a plurality of peptides comprising protecting groups, each peptide immobilized on a solid support, optionally:

    a) further comprising exposing a deprotection reagent to the immobilized peptides to remove the protecting groups from at least a portion of the immobilized peptides and removing at least a portion of the deprotection reagent, and/or
    b) further comprising exposing activated amino acids to the immobilized peptides such that at least a portion of the activated amino acids are bonded to the immobilized peptides to form newly-bonded amino acid residues, and optionally removing at least a portion of activated amino acids that do not bond to the immobilized peptides, and/or

c) wherein the protecting groups comprise fluorenylmethyloxycarbonyl protecting groups and/or tert-butyloxycarbonyl protecting groups.

**Patentansprüche**

1. Verfahren zur Inbetriebnahme eines Peptidsynthesesystems, umfassend:

> Starten des Flusses eines ersten Fluidstroms, umfassend Aminosäuren, von einem ersten Reagensreservoir zu einem Mischbereich,
> Starten des Flusses eines zweiten Fluidstroms, umfassend eine Base, von einem zweiten Reagensreservoir zu dem Mischbereich so, dass eine Vorderkante des ersten Fluidstroms und eine Vorderkante des zweiten Fluidstroms den Mischbereich innerhalb von etwa 10 ms erreichen,
> gegebenenfalls Starten des Flusses eines dritten Fluidstroms, umfassend ein Aktivierungsmittel,
> Vereinigen des ersten und zweiten Fluidstroms und gegebenenfalls des dritten Fluidstroms in einem Mischbereich zur Bildung eines Mischfluidstroms und
> Fließen des Mischfluidstroms zu einem Reaktor.

2. Verfahren gemäß Anspruch 1, umfassend Starten des Flusses des dritten Fluidstroms, umfassend das Aktivierungsmittel, und Vereinigen des ersten, zweiten und dritten Fluidstroms in dem Mischbereich zur Bildung des Mischfluidstroms.

3. Verfahren zur Inbetriebnahme eines Peptidsynthesesystems, umfassend:

> Starten des Flusses eines ersten Fluidstroms, umfassend ein Aktivierungsmittel, von einem ersten Reagensreservoir zu einem Mischbereich,
> Starten des Flusses eines zweiten Fluidstroms, umfassend eine Base, von einem zweiten Reagensreservoir zu dem Mischbereich so, dass eine Vorderkante des ersten Fluidstroms und eine Vorderkante des zweiten Fluidstroms den Mischbereich innerhalb von 10 ms erreichen,
> gegebenenfalls Starten des Flusses eines dritten Fluidstroms, umfassend Aminosäuren,
> Vereinigen des ersten und zweiten Fluidstroms und gegebenenfalls des dritten Fluidstroms in einem Mischbereich zur Bildung eines Mischfluidstroms und
> Fließen des Mischfluidstroms zu einem Reaktor.

4. Verfahren gemäß Anspruch 3, umfassend Starten des Flusses des dritten Fluidstroms, umfassend die Aminosäuren, und Vereinigen des ersten, zweiten und dritten Fluidstroms in dem Mischbereich zur Bildung des Mischfluidstroms.

5. Verfahren gemäß irgendeinem vorhergehenden Anspruch, umfassend das Fließen eines vierten Fluidstroms, umfassend ein Additiv, ausgewählt aus der Gruppe, bestehend aus einem chaotropen Salz, einem Co-Lösungsmittel und einem Tensid, und ferner umfassend das Vereinigen des ersten, zweiten, dritten und vierten Fluidstroms in dem Mischbereich.

6. Verfahren gemäß irgendeinem vorhergehenden Anspruch, worin:

> a) ein Molverhältnis der Aminosäuren zu der Base, gemessen an der Vorderkante, wenn die Vorderkante in den Reaktor eintritt, innerhalb von 10 % eines Molverhältnisses der Base zu dem Aktivierungsmittel in dem Mischfluidstrom im Mischbereich liegt und/oder
> b) ein Molverhältnis der Base zu dem Aktivierungsmittel, gemessen an der Vorderkante, wenn die Vorderkante in den Reaktor eintritt, innerhalb von 10 % eines Molverhältnisses der Base zu dem Aktivierungsmittel in dem Mischfluidstrom im Mischbereich liegt und/oder
> c) ein Molverhältnis der Aminosäuren zu der Base, gemessen an der Vorderkante, wenn die Vorderkante in den Reaktor eintritt, innerhalb von 10 % eines Molverhältnisses des Aktivierungsmittels zu der Base in dem Mischfluidstrom am Eingang zum Reaktor zu einer Zeit liegt, die mindestens etwa 10 ms, nachdem die Vorderkante in den Reaktor eintritt, liegt, und/oder
> d) ein Molverhältnis der Base zu dem Aktivierungsmittel, gemessen an der Vorderkante, wenn die Vorderkante in den Reaktor eintritt, innerhalb von 10 % eines Molverhältnisses des Aktivierungsmittels zu der Base in dem Mischfluidstrom am Eingang zum Reaktor zu einer Zeit liegt, die mindestens etwa 10 ms nachdem die Vorderkante in den Reaktor eintritt, liegt.

7. Verfahren gemäß irgendeinem vorhergehenden Anspruch, worin ein Molverhältnis der Aminosäuren zu der Base im Mischfluidstrom mehr als etwa 1:1 beträgt.

8. Verfahren gemäß irgendeinem vorhergehenden Anspruch, worin das Aktivierungsmittel aus der Gruppe, bestehend aus einem Carbodiimid, Guanidiniumsalz, Phosphoniumsalz und Uroniumsalz, ausgewählt ist.

9. Verfahren gemäß irgendeinem vorhergehenden Anspruch, worin:

a) die Base eine Lewis-Base ist und/oder
b) die Base eine nicht-nukleophile Base ist.

10. Verfahren gemäß irgendeinem vorhergehenden Anspruch, worin:

a) der Mischfluidstrom keiner Wärme aus einer Wärmequelle vor Erreichen des Reaktors ausgesetzt wird und/oder
b) der Mischfluidstrom keiner Wärme aus einer Wärmequelle zwischen dem Mischbereich und dem Eingang zum Reaktor ausgesetzt wird.

11. Verfahren gemäß irgendeinem vorhergehenden Anspruch, worin:

a) die Temperatur des Mischfluidstroms innerhalb von 10°C der Temperatur der Vorderkante am Eingang zum Reaktor liegt und/oder
b) die Temperatur der Vorderkante um weniger als 10°C vom Mischbereich bis zum Eingang zum Reaktor variiert.

12. Verfahren gemäß irgendeinem vorhergehenden Anspruch, worin der Reaktor keine Vielzahl von Aminosäuren und/oder keine Vielzahl von Peptiden umfasst, die auf einem festen Träger immobilisiert sind.

13. Verfahren gemäß irgendeinem der Ansprüche 1-12, worin:
der Reaktor eine Vielzahl von auf einem festen Träger immobilisierten Peptiden umfasst, gegebenenfalls worin:

a) der feste Träger in einer gepackten Säule und/oder einem Fließbett enthalten ist und/oder der feste Träger ein Harz, ein mikroporöses Polystyrolharz, ein mikroporöses Polyethylenglykolharz und/oder ein mikroporöses Copolymerharz umfasst und/oder
b) das Verfahren ferner das Aussetzen der Aminosäuren der immobilisierten Peptide so, dass mindestens ein Teil der Aminosäuren an die immobilisierten Peptide zur Bildung von verlängerten Peptiden gebunden werden, umfasst und/oder
c) mehr als etwa 99 % der immobilisierten Peptide während des Aussetzungsschritts jeweils an einem einzigen Aminosäuremolekül gebunden werden.

14. Verfahren gemäß irgendeinem vorhergehenden Anspruch:
ferner umfassend das Bereitstellen einer Vielzahl von Peptiden, die Schutzgruppen umfassen, wobei jedes Peptid auf einem festen Träger immobilisiert ist, gegebenenfalls:

a) ferner umfassend das Aussetzen eines Entschützungsreagenzes den immobilisierten Peptiden zur Entfernung der Schutzgruppen von mindestens einem Teil der immobilisierten Peptide und das Entfernen von mindestens einem Teil des Entschützungsreagenzes und/oder
b) ferner umfassend das Aussetzen von aktivierten Aminosäuren den immobilisierten Peptiden so, dass mindestens ein Teil der aktivierten Aminosäuren an die immobilisierten Peptide zur Bildung von neu gebundenen Aminosäureresten gebunden werden, und gegebenenfalls Entfernen von mindestens einem Teil der aktivierten Aminosäuren, die nicht an die immobilisierten Peptide binden, und/oder
c) wobei die Schutzgruppen Fluorenylmethyloxycarbonyl-Schutzgruppen und/oder tert-Butyloxycarbonyl-Schutzgruppen umfassen.

**Revendications**

1. Procédé de mise en fonctionnement d'un système de synthèse de peptide, comprenant:

le déclenchement d'un écoulement d'un premier courant de fluide comprenant des acides aminés d'un premier réservoir de réactif à une région de mélange ;
le déclenchement d'un écoulement d'un deuxième courant de fluide comprenant une base d'un second réservoir de réactif à la région de mélange, de sorte qu'un bord d'attaque du premier courant de fluide et qu'un bord d'attaque du deuxième courant de fluide arrivent à la région de mélange environ au sein de 10 ms l'un de l'autre ;
facultativement le déclenchement d'un écoulement d'un troisième courant de fluide comprenant un agent d'activation ;
la convergence des premier et deuxième courants de fluide, et facultativement du troisième courant de fluide, au niveau d'une région de mélange pour former un courant de fluide mélangé ; et
l'écoulement du courant de fluide mélangé vers un réacteur.

2. Procédé selon la revendication 1, comprenant le déclenchement de l'écoulement du troisième courant de fluide comprenant l'agent d'activation et la convergence des premier, deuxième et troisième courants de fluide au niveau de la région de mélange pour former le courant de fluide mélangé.

3. Procédé de mise en fonctionnement d'un système de synthèse de peptide, comprenant :

le déclenchement d'un écoulement d'un premier courant de fluide comprenant un agent d'activation d'un premier réservoir de réactif à une région de mélange ;

le déclenchement d'un écoulement d'un deuxième courant de fluide comprenant une base d'un second réservoir de réactif à la région de mélange, de sorte qu'un bord d'attaque du premier courant de fluide et qu'un bord d'attaque du deuxième courant de fluide arrivent à la région de mélange environ au sein de 10 ms l'un de l'autre ;

facultativement le déclenchement d'un écoulement d'un troisième courant de fluide comprenant des acides aminés ;

la convergence des premier et deuxième courants de fluide, et facultativement du troisième courant de fluide, au niveau d'une région de mélange pour former un courant de fluide mélangé; et

l'écoulement du courant de fluide mélangé vers un réacteur.

**4.** Procédé selon la revendication 3, comprenant le déclenchement de l'écoulement du troisième courant de fluide comprenant les acides aminés et la convergence des premier, deuxième, et troisième courants de fluide au niveau de la région de mélange pour former le courant de fluide mélangé.

**5.** Procédé selon une quelconque revendication précédente, comprenant l'écoulement d'un quatrième courant de fluide comprenant un additif sélectionné parmi le groupe consistant en un sel chaotropique, un cosolvant et un tensioactif, et comprenant en outre la convergence des premier, deuxième, troisième et quatrième courants de fluide au niveau de la région de mélange.

**6.** Procédé selon une quelconque revendication précédente, dans lequel :

a) un rapport molaire entre les acides aminés et la base mesuré au niveau du bord d'attaque lorsque le bord d'attaque pénètre dans le réacteur ne diffère pas de plus de 10 % d'un rapport molaire entre la base et l'agent d'activation dans le courant de fluide mélangé au niveau de la région de mélange ; et/ou

b) un rapport molaire entre la base et l'agent d'activation mesuré au niveau du bord d'attaque lorsque le bord d'attaque pénètre dans le réacteur ne diffère pas de plus de 10% d'un rapport molaire entre la base et l'agent d'activation dans le courant de fluide mélangé au niveau de la région de mélange ; et/ou

c) un rapport molaire entre les acides aminés et la base mesuré au niveau du bord d'attaque lorsque le bord d'attaque pénètre dans le réacteur ne diffère pas de plus de 10% d'un rapport molaire entre l'agent d'activation et la base dans le courant de fluide mélangé à l'entrée du réacteur à un moment qui a lieu au moins environ 10 ms après que le bord d'attaque pénètre dans le réacteur ; et/ou

d) un rapport molaire entre la base et l'agent d'activation mesuré au niveau du bord d'attaque lorsque le bord d'attaque pénètre dans le réacteur ne diffère pas de plus de 10% d'un rapport molaire entre l'agent d'activation et la base dans le courant de fluide mélangé à l'entrée du réacteur à un moment qui a lieu au moins environ 10 ms après que le bord d'attaque pénètre dans le réacteur.

**7.** Procédé selon une quelconque revendication précédente, dans lequel un rapport molaire entre les acides aminés et la base dans le courant de fluide mélangé est supérieur à environ 1:1.

**8.** Procédé selon une quelconque revendication précédente, dans lequel l'agent d'activation est sélectionné parmi le groupe consistant en un carbodiimide, un sel de guanidinium, un sel de phosphonium et un sel d'uronium.

**9.** Procédé selon une quelconque revendication précédente, dans lequel :

a) la base est une base de Lewis ; et/ou
b) la base est une base non nucléophile.

**10.** Procédé selon une quelconque revendication précédente, dans lequel :

a) le courant de fluide mélangé n'est pas exposé à la chaleur provenant d'une source de chaleur avant son arrivée au réacteur ; et/ou
b) le courant de fluide mélangé n'est pas exposé à la chaleur provenant d'une source de chaleur entre la région de mélange et l'entrée dans le réacteur.

**11.** Procédé selon une quelconque revendication précédente, dans lequel :

a) la température du courant de fluide mélangé ne diffère pas de plus de 10 °C de la température du bord d'attaque à l'entrée du réacteur ; et/ou
b) la température du bord d'attaque varie de moins de 10 °C de la région de mélange à l'entrée du réacteur.

**12.** Procédé selon une quelconque revendication précédente, dans lequel le réacteur ne comprend pas une pluralité d'acides aminés et/ou une pluralité de peptides immobilisés sur un support solide.

13. Procédé selon l'une quelconque des revendications 1-12, dans lequel :
le réacteur comprend une pluralité de peptides immobilisés sur un support solide, facultativement dans lequel :

a) le support solide est contenu à l'intérieur d'une colonne remplie et/ou d'un lit fluidisé, et/ou le support solide comprend une résine, une résine de polystyrène microporeuse, une résine de polyéthylène glycol microporeuse et/ou une résine de copolymère microporeuse ; et/ou

b) le procédé comprend en outre l'exposition des acides aminés aux peptides immobilisés de sorte qu'au moins une portion des acides aminés soit liée aux peptides immobilisés pour former des peptides allongés ; et/ou

c) plus de 99 % environ des peptides immobilisés deviennent chacun lié à une seule molécule d'acide aminé pendant l'étape d'exposition.

14. Procédé selon une quelconque revendication précédente :
comprenant en outre la fourniture d'une pluralité de peptides comprenant des groupes protecteurs, chaque peptide étant immobilisé sur un support solide, facultativement :

a) comprenant en outre l'exposition d'un réactif de déprotection aux peptides immobilisés pour éliminer les groupes protecteurs d'au moins une portion des peptides immobilisés et l'élimination d'au moins une portion du réactif de déprotection, et/ou

b) comprenant en outre l'exposition d'acides aminés activés aux peptides immobilisés de sorte qu'au moins une portion des acides aminés activés soit liée aux peptides immobilisés pour former des résidus d'acides aminés nouvellement liés, et facultativement l'élimination d'au moins une portion des acides aminés activés qui ne se lient pas aux peptides immobilisés, et/ou

c) dans lequel les groupes protecteurs comprennent des groupes protecteurs fluorénylméthyloxycarbonyle et/ou des groupes protecteurs tert-butyloxycarbonyle.

FIG. 1

FIG. 1 cont'd

FIG. 2A

FIG. 2B

# FIG. 3A

GCβRPRGDNPPLβCKQDSDCLAGCVCGPNGFCG

# FIG. 3B

Non-Synchronized Concentration     Synchronized Concentration

—— Reagent A    —— Reagent B      —— Reagent A    —— Reagent B

FIG. 3C

ACP – VQAAIDYING
70°C, HATU

20 mL/min

40 mL/min

60 mL/min

Counts (%) vs. Acquisition Time (min)

FIG. 4A

30

FIG. 4B

FIG. 5A

| | Couple | Wash | Deprotect | Wash |
|---|---|---|---|---|
| Method A 40 mL/min 70°C | 0.17 M HATU 0.2 M AA 5 v/v% DIEA 9.6 mL | DMF 16.8 mL | 20% (v/v) piperidine in DMF 11.2 mL | DMF 12.6 mL |
| Method B 80 mL/min 90°C | Couple | Wash | Deprotect | Wash |

FIG. 5B

FIG. 5C

FIG. 5D

FIG. 6A

## Insulin B-Chain

FVNQH LCGSH LVEAL YLVCG ERGFF YTPKT-CONH₂

i. Automated Flow Synthesis
   Method B
   20 minutes

ii. Manual Batch Synthesis
   30 hours

Total Ion Counts vs. Time (min)

## FIG. 6B

## Deprotection Data from Automated Syntheses

GHRH

Insulin B Chain

% of First Deprotection

Area
Width at Half Maximum
Height

Coupling Number

## FIG. 6C

FIG. 7A

FIG. 7B

FIG. 7C

FIG. 7D

FIG. 7E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014149387 A2

**Non-patent literature cited in the description**

- *Letters in Peptide Science,* 2002, vol. 9, 203-206

- **KENT et al.** *Org Lett,* 2015, vol. 17 (14), 3521